# EUROPEAN PATENT SPECIFICATION

(11) **EP 0 942 741 B1**
(45) Date of publication and mention of the grant of the patent: **11.12.2002**
(21) Application number: 97927282.0
(22) Date of filing: 20.06.1997
(51) Int. Cl.: A61K 38/16, A23L 1/305

(54) **LECTIN COMPOSITIONS AND USES THEREOF**
LEKTINZUSAMMENSTELLUNGEN UND DEREN VERWENDUNG
COMPOSITIONS DE LECTINE ET UTILISATION DE CES COMPOSITIONS

(30) Priority: 21.06.1996 GB 9613070
(43) Date of publication of application: 22.09.1999
(73) Proprietor: Alizyme Therapeutics Limited, Cambridgeshire CB1 6GX (GB)
(72) Inventor: PUSZTAI, Arpad Janos, Rowett Research Inst., Bucksburn, Aberdeen AB2 9SBB (GB); BARDOCZ, Zsuszanna Magdolna, Rowett Research Inst., Bucksburn, Aberdeen AB2 9SB (GB); PALMER, Richard Michael John, Alizyme Therapeutics, Cambridge CB4 4WE (GB); FISH, Neil William, Alizyme Therapeutics Limited, Cambridge CB4 4WE (GB); KOTELES, Gyorgy J., Frederic Jolio-Curie, H-1221 Budapest (HU)
(74) Representative: Cornish, Kristina Victoria Joy
(86) International application number: GB9701668
(87) International publication number: WO97049420

(56) References cited:
- DE-A- 4 318 307
- US-A- 5 053 386
- PUSZTAI, A. ET AL: "Dietary lectins as extraneous metabolic signals" LECTINS: BIOL., BIOCHEM., CLIN. BIOCHEM. (1994), 9, 131-138, 1994, XP002040875
- BARDOCZ S ET AL: "The effect of phytohaemagglutinin at different dietary concentrations on the growth, body composition and plasma insulin of the rat." BRITISH JOURNAL OF NUTRITION 76 (4). 1996. 613-626, XP002040876

## Description

The invention relates to use of lectins in the manufacture of medicaments for the control of mucosal cell proliferation, for the reduction and/or treatment of damage caused by a cell-damaging agent.

Lectins are proteins or glycoproteins, typically of plant or even microbial or animal origin which recognise and attach to specific glycoconjugate structures. Orally administered lectins such as kidney bean (*Phaseolus vulgaris*) lectin, phytohaemagglutinin (PHA), can be powerful extraneous growth factors for the rat gut, inducing fully reversible, polyamine-dependent, hyperplastic growth of the small intestine (Bardocz et al., 1992). The lectin avidly binds to the brush border and is partially transcytosed into the circulation (Pusztai, 1991). At particular doses, lectins such as PHA damage the gut wall, causing coliform overgrowth in the lumen (Pusztai et al., 1993), increasing the rate of lipid mobilisation and glucose oxidation (Grant et al., 1987) and significantly reducing the fractional rate of protein synthesis in skeletal muscle (Palmer et al., 1987; Bardocz et al., 1992). Thus, lectins such as PHA are generally regarded as nutritional toxins because at high oral doses they induce severe losses of body lipids, glycogen and muscle protein (Pusztai, 1991) and possibly death.

Safe, non-toxic threshold levels for the oral administration of lectins in human and other animals are not known.

Mucosal cells are those which make up any mucous membrane (the moist membrane lining many tubular structures). Many are cells which provide a protective layer between the external environment and the internal organs of an animal. Examples of mucosal cells include the epithelial cells of the skin, the lining of the alimentary canal, the tissue covering the eye and the lining of the lung and nose. A number of disorders of mucosal cells are known, including conditions where cell division is accelerated, decelerated, where the mucosal cells are damaged, or the protective outer layer such as mucous is missing. Conditions related to abnormal control of mucosal cell proliferation may include skin cancers, psioriasis, irritable bowel syndrome, inflammatory bowel disease and mucositis. Mucositis is a painful and debilitating condition in which the rapidly growing epithelial and mucosal cells are damaged and the external mucous layer is removed and/or not replaced sufficiently quickly, Mucositis may result in infection by microorganisms which are present, for example, in the mouth or gut. The condition is seen as a major side effect in the treatment of cancer. The incidence and severity of mucositis may increase with increasing rounds of cancer therapy, and may ultimately affect patient treatment compliance and survival.

Agents which damage mucosal (or other) cells and which may cause mucositis include, chemotherapeutic agents, radiotherapy, chemicals (organic or inorganic), toxins, acids, alkali, any radiation source and free radicals. Chemotherapy and radiotherapy, used either alone, used together or in combination with surgery are the main therapeutic approaches for the treatment of cancer. Chemotherapy may use a cytotoxic agent to directly damage the DNA of a target cell. If a sufficient dose of the cytotoxic agent is administered to a target tissue i.e. a tumour, DNA mis-repair may result in the accumulation of DNA mutations, lesions and chromosomal aberrations that ultimately result in cell death. Radiotherapy uses radiation to either damage the DNA of a target cell directly, or exploits the potential of ionising radiation to produce free radicals which are able to break DNA strands (Steel, 1996).

The principle by which chemotherapy is used to treat cancer is that a cytotoxic drug is administered to inhibit cell division, which may ultimately lead to cell death. As the cancerous cells are usually growing more rapidly than normal tissue, the expectation is that the cytotoxic drug will kill more cancerous cells than normal cells. Unlike radiotherapy however, the cytotoxic drugs are given in such a manner that they act systemically throughout the body. Serious side effects, such as toxicity to vital tissues including bone marrow may limit the dose of cytotoxic drug that can be administered without killing the patient In a similar manner, the use of radiation to treat cancer does not discriminate between cancerous and normal tissue. The use of radiotherapy is therefore a compromise in trying to induce most damage to the cancerous cells by targeting the radiation without irreparably damaging normal tissue.

Many cytotoxic drugs have been developed and evaluated for the treatment of cancer. The main principle by which these drugs act is that they interfere or will inhibit key steps in the cell division pathway. The major drug classes target either DNA replication, DNA repair, chromosome separation or cytoplasmic division. The vast majority of cytotoxic drugs interfere with the synthesis and replication of DNA. 5-fluorouracil (5-FU) is one of the most commonly used cytotoxic drugs in this class. The activity of 5-FU can also be modulated by the addition of reduced folates such as calcium leucovorin (Isacoff et al, 1994). Other cytoxic drugs that inhibit DNA synthesis and replication are known which target different deoxyribonucleotides used to make DNA e.g. cytarabine. DNA strands containing cytarabine directly inhibit the activity of DNA polymerase (Archimund & Thomas, 1994).

A second major class of cytotoxic drugs are those which induce the breakage of DNA strands directly, or those which inhibit the repair of DNA breaks. Cyclophosphamide is an example of a drug that can break DNA strands directly (Sparano & Wiernik, 1994). A third major class of drugs are those that actually disrupt the assembly and disassembly of tubulin, so inhibiting mitosis and cell division directly. Taxanes such as paclitaxel and docetaxel are drugs which polymerise tubulin into stable microtubule bundles. Synthetic vinca-alkaloids such as vinorelbine are spindle poisons which exert their anti-tumour effects by preventing the assembly of tubulin into microtubules (Dieras & Pouillart, 1995).

Current radiotherapy practice uses a range of radiation sources to treat cancer. The most commonly used sources are X-ray, gamma ray, proton or neutron sources of α or β emitters. In practice, continuous low dose radiotherapy over several days gives the best chances of discriminating between normal and cancerous tissue. However, this technique is limited to the use of radio-isotopes which are currently only effective with a few tumour types, e.g. thyroid cancer. In the clinical situation, most radiotherapy techniques use high doses of radiation which are focused as a beam at the cancerous tissue. Exposure of normal tissue is reduced, where possible, by the use of lead shielding or by rotating the patient such that normal tissue receives a lower dose than the cancerous cells. Although this approach can be effective, its use may be limited by cumulative exposures of normal tissue to radiation and the resistance of many tumours to high doses of radiation.

It is well known in the art that single cytotoxic agents or radiation sources may be more suitable for certain cancer types. For example, Cisplatin is widely used for testicular cancer, taxanes are more suitable for breast cancer and 5-FU is widely used for colorectal cancers. However, single agent therapies rarely provide a complete cure for cancer and rates of survival are still low. Some improvements have been made in the use of multiple drug regimes (Au et al, 1996).

A number of compounds have been evaluated in the art for their ability to sensitise cancer cells to the effects of radiation and chemotherapy (so sparing normal tissue). However, the use of radiosensitisers such as the vitamin K mimetics Synkavit and Menadione and protectants such as the sulphidryl containing compounds cysteine, cysteamine and Ethyol have also been disappointing (Denekamp, 1996).

Although chemotherapy and radiotherapy are the most widely used treatments for cancer, the rates of survival are limited due to a number of factors. The key factor is that the cytotoxic drug or radiation does not discriminate between normal and cancerous tissue. In most cases, it is impossible to give a sufficient dose of cytotoxic drug or radiation to reliably kill all cancerous tissue as it would prove fatal to the patient. Common side effects for existing therapy regimes include hair loss, bone marrow suppression, nausea, vomiting and diarrhoea (Paulsen *et al* 1996). In addition, there are also many instances where the use of radiotherapy, particularly to the pelvic regions has resulted in altered gastrointestinal function (Yeoh *et al* 1993) and long term damage to the gut which requires surgery (van Halteren *et al* 1993).

A major breakthrough has been made in the last 10 years with the availability of hematopoietic growth factors. It is now possible to give higher doses of cytotoxic drugs and radiation and then rescue tissues such as bone marrow and white blood cells by the administration of recombinant growth factors such as granulocyte-macrophage colony-stimulating factor (Erkisis et al, 1996). Such an approach has enabled improved prognosis and survival rates to be achieved. Whilst epidermal-specific growth factors such as epidermal growth factor are known, the complex nature of gut growth regulation has made it difficult to develop effective gut "rescue" procedures (Podolsky, 1993). As no such growth factor currently exists for the gut, damage to the gastrointestinal tract by cytotoxic drugs and radiation has now become dose limiting.

The present invention utilises the tissue protecting qualities and the metabolic effects of low doses of lectins to protect and repair biological material damaged by radiotherapy and/or chemotherapy. The present invention is of particular interest because of the noted prophylactic effects of lectin compositions (positive growth factors) before treatments such as radiotherapy and/or chemotherapy.

Accordingly, the present invention provides, as a first aspect, the use of a lectin in the manufacture of a medicament for the control of mucosal cell proliferation. The control of mucosal cell proliferation includes the reduction and/or treatment of any damage to mucosal cells and/or tissues. Throughout this text, "reduction" means any effect which mitigates any damage or any medical disorder, to any extent, and includes prevention. Throughout this text, "treatment" means any amelioration of disorder, disease, syndrome, condition, pain or a combination of two or more thereof.

In particular, the first aspect of the invention pertains to the control of mucosal cell proliferation in the reduction and/or treatment of mucositis and/or gut lesions.

Control of mucosal cell proliferation is particularly useful in the reduction and/or treatment of damage caused by a cell-damaging agent. Typical cell damaging agents of all aspects of the present invention include radiotherapy, chemotherapy or a combination thereof. In the present application the terms "irradiation" and "radiotherapy" are used as having the same meaning, which is a source of irradiation which may, or may not be applied as a therapeutic technique.

The first aspect of the present invention is particularly effective in the control of mucosal cell proliferation prior to, during or following radiotherapy, chemotherapy or a combination thereof. The invention is achieved as a result of the protective and repair capabilities of lectins.

Mucosal cells are those which make up any mucous membrane (the moist membrane lining many tubular structures and cavities). Many are those which provide a protective layer between the external environment and the internal organs of an animal. Mucosal cells/tissues include cells of the nasal sinuses, the respiratory tract, the skin, the gastrointestinal tract as well as biliary and pancreatic systems. The surface of the mouth is also lined by a mucous membrane. The mucous membrane consists of a surface layer of epithelium, which contains glands secreting mucous, with underlying layers of connective tissue and muscularis mucosae, which forms the inner boundary of the mucous membrane.

The use of the lectin for the control of mucosal cell proliferation is particularly useful in relation to cells of the gastrointestinal tract. The control may be for an increase in functional and/or length of the gastrointestinal tract or for control of the nature and/or density of gastrointestinal-cell expressed surface glycoproteins. Other uses in relation to control of mucosa cell proliferation include the reduction and/or treatment of bowel disorders such as inflammatory bowel disease and irritable bowel syndrome as well as the reduction and/or treatment of gut lesions, and the repair and replacement of mucosa cells prior to, during or following, radiotherapy, chemotherapy or a combination of two or more thereof.

The applications of these control features includes:
(a) Gut cell proliferation leading to an increase in functional gut area and length which could be a useful therapy where gut function is impaired as for instance via surgery or accident, and or
(b) Control of gut cell turnover rates which may allow control over the nature and density of expressed surface glycoconjugates as these become progressively more complex as cells age. As the glycoconjugates affect certain gut properties, such as propensity to bacterial attachment, lectin administration could be envisaged as a therapeutic or prophylactic control for these properties.

In particular, the control of cell proliferation may be used for obtaining an increased nutrient capacity of the small intestine and/or controlling glycoconjugate expression of gut cells. Such effects are not necessarily medical disorders and may be only cosmetic or functional, above and beyond the satisfactory medical level.

According to a second aspect of the invention, there is provided the use of a lectin in the manufacture of a medicament for the reduction and/or treatment of damage caused by a cell-damaging agent. Cell-damaging agents include radiotherapy, chemotherapy or a combination thereof. Damage includes gut lesions and/or mucositis, in particular.

For the first and second aspects of the invention, sources of radiotherapy include, but are not limited to, X-ray, gamma ray, proton or neutron sources, α or β emitters or a combination of two or more thereof. The radiotherapy may be used in combination with chemotherapy using a cytotoxic agent such as methotrexate, Cisplatin and/or 5-fluorouracil, and also in combination with surgical procedures.

For the first and second aspects of the invention, chemotherapeutic agents include any cytotoxic agent and include, but are not limited to agents such as 5-fluorouracil, Cisplatin, doxorubicin, methotrexate, a taxol or a combination of two or more thereof. As described above, one or more chemotherapeutic agent can be used in combination with radiotherapy and/or surgical procedures.

The invention is of particular applicability to the reduction and/or treatment of damage to mammalian tissue, more particularly human tissue. The present invention is of particular importance to human tissue because of the considerable requirement for radiotherapy and/or chemotherapy in the treatment of cancer. However, the present invention is also applicable to the veterinary industry, including farm animals and pets.

The first aspect of the invention relates to all mucosal cells and/or tissues, and includes mucosal cells and tissues within a whole body as well as outside of a whole body, including isolated mucosal cells and tissues. The second aspect of the invention relates to all biological matter, including whole bodies and parts thereof, and includes isolated organs and isolated tissue, including mucosal cells and tissues. The invention also relates to matter which is subjected, intentionally or unintentionally, to any cell-damaging agent.

The invention is of particular use to biological matter which is particularly sensitive to cell-damaging agents such as radiotherapy and/or chemotherapeutic agents. Such biological matter according to the first aspect of the invention includes the mucosal coverings of the gut, the mouth, the nasal passage, the oesophagus, the stomach, the lung, the small intestine, the large intestine (including the colon and the rectum), epithelial tissue (eg covering the eye) as well as any other mucosal cell and/or tissue.

Such biological matter according to the second aspect of the invention includes all of the mucosal cells and tissues defined above, as well as bone marrow, spleen, all blood generating cells, blood tissue, thymus, hair-producing tissue, eye tissue and testicular/prostate tissue. The sensitivity of the gut to damage by a cell-damaging agent is linked to its metabolic status. It is the growth factor effect of lectins, in particular on the gut (and especially the small intestine) which is understood to be important in the prophylactic and/or therapeutic affects prior to and during administration of cell-damaging agents.

Lectins can be classified as either "toxic" or "non-toxic". Toxic lectins include or may be classified as type 2 Ribosome Inactivating Proteins (RIPs). These are hybrid molecules which contain a toxic (A) subunit which, after entering the cell, irreversibly inhibits protein synthesis and a lectin subunit (B) which facilitates the entry of RIP into the cell. Type 2 RIPs such as Ricin are some of the most poisonous substances known to man with potential LD₅₀ values as low as 0.1g per kg body weight They can irreversibly damage a mammal on long term administration, ultimately leading to death.

The binding of lections to cells varies greatly. Some bind weakly and for others the binding is very strong. Strongly binding describes a lectin wherein if 10mg is administered orally to a rat, over 75% (and up to 100%) binds to the gut. Kidney bean lectin is an example of a strongly binding lectin. In some instances strong binding may lead to toxicity, and these lectins may be referred to as toxic.

The lectins of the present invention include those either in naturally occurring compositions or purified therefrom (to any extent), chemically synthesised lectins, modified lectins or derivatives (naturally occurring or synthetic) thereof. Derivatives of lectins include one or more subunits of multi-subunit lectins. Methods are well known in the art for the production of lectins and include, the purification of lectins from natural sources (Pusztai and Palmer, 1977: Carvalho, 1993) and biotechnology-derived lectins such as described in US patent no. 4,889,842.

Any lectin can be used according to the first aspect of the invention. Many lectins are known. A widely used method for characterising lectins is their carbohydrate binding specificity. A number of the carbohydrate binding specific groups include: N-acetyl-D-galactosamine, -D-mannose, -L-fucose, beta-lactose, Galactosyl-beta-(1-3)-N-acetyl-D-galactosamine, D-glucose, N-acetyl-glucosamine, N-acetyl-neuraminic acid. Some lectins fall within more than one carbohydrate binding specific group. Of particular interest for the present invention are lectins from; kidney bean, soya bean, Jack bean, wheat germ, lotus seed, onion, lentil, tomato, potato and combinations of two or more thereof.

Since lectins are proteins, they are clearly subject to destabilisation/denaturation by a number of parameters such as heat, acid, alkali etc. Some lectins are more resistant to these effects than others. Since the use of lectins in the present invention (throughout) requires their protein properties it is important that the lectins are not completely destroyed or denatured prior to or during their use (eg in the strong acid conditions of the stomach and the mild alkali conditions of the lower gut). Accordingly, lectins for use in the present invention may need to be first characterised with regard to how they may be affected during processing and/or administration. Such characterisation is standard technology and can be conducted easily by any person skilled in the art. The concentration of lectin required for any of the aspects of the invention will vary if the lectin has been denatured or destabilised in any way.

The concentrations of lectins which have been provided in this text are based on their natural properties with effectively no reduction of their activity by denaturation or destabilisation. Thus, the concentrations of lectins which are provided are not absolute but reflect the activity of the lectin. Accordingly, a composition, for example, which has a lectin whose activity has been reduced by half, but which is present in double the concentration is the same as a composition which has half the quantity of lectin but where the activity of the lectin has not been altered. Furthermore, the activity of any lectin may be increased by modification, for example during recombinant production and/or by producing truncated mutants which have increased activity. The same considerations as to the concentration verses activity of lectin also applies to lectins with increased activity. All modified lectins are covered by the present invention, including those with increased or decreased activities, and include, for example, truncated lectin monomers with full or modified activity.

The lectins according to the present invention are tissue protectants.

The first and second aspects of the invention relate to the manufacture of medicaments, the particular dosage regime for which will ultimately be determined by the attending physician and will take into consideration such factors as the lectin or lectins being used; animal type, age, weight, severity of symptoms and/or severity of treatment being or to be applied, method of administration of the composition, adverse reactions and/or other contraindications. Specific defined dosage ranges can be determined by standard designed clinical trials with patient progress and recovery being fully monitored. Such trials may use an escalating dose design using a low percentage of the maximum tolerated dose in animals as the starting dose in man. Preliminary guidance for dosage ranges can be taken from the results given in the experimental section of this text and by the following ranges which have been extrapolated to humans.

An appropriate upper limit appears to be a concentration of a lectin up to approximately 0.3 g per kg body weight per day. A concentration of 0.3 g per kg body weight per day may well produce an upset gastro-intestinal tract in a patient but these symptoms may be acceptable on the basis that the patient has an increase chance of survival from diseases such as cancer. An appropriate lower limit appear to be a concentration of lectin around 0.0001 mg (0.1 µg) per kg body weight per day. Preferred intermediate dosage concentrations include the presence of a lectin up to approximately 0.2 g, 0.15 g and 0.05 g per kg body weight per day, and thereafter of concentrations approximately 1 mg, 0.5 mg, 0.1 mg, 0.01 mg and 0.005 mg per kg body weight per day. Any of these given concentrations can be used as an upper and/or low limit, thus providing a variety of useful ranges of concentration. With regard to concentrations of lectins for administration, when not used for a therapeutic or prophylactic effect the dose of lectin may be slightly raised.

The medicaments for all aspects of the present invention can be used to provide the required concentration of lectin in one or more "intake" per day ("intake" including any form of administration). Some aspects of the invention may suit a single intake of a high lectin concentration, whereas others may suit a number of intakes, evenly or unevenly spaced over the same period, but with a lower lectin concentration per intake or divided doses.

Use of the lectin in the manufacture of any medicament according to the present invention may be in combination with a suitable pharmaceutical carrier and/or excipient. Such carriers and excipients are well known in the art (eg Handbook of Pharmaceutical Excipients (1994) 2^{nd} Edition, Eds. A. Wade/P.J. Weller, The Pharmaceutical Press, American Pharmaceutical Association). Of particular use according to the present invention are compositions and/or medicaments which are formulated in a colonic drug delivery system or in a capsule.

The first and second aspects of the invention also cover the use of a lectin in combination with a cytoprotectant. The cytoprotectant is preferably a radiosensitiser, a chemoprotectant (including free-range scavangers), a growth factor or a combination of two or more thereof. From the above list of cytoprotectants the following examples are preferred: radiosensitisers- vitamin K mimetics such as Synkavit or Menadione, gadolinium texaphyrin or iobenguane (([[3-iodo-13311)phenyl]methyl]guanidine): chemoprotectants- Sulcraphate, cysteine, cysteamine, Ethyol, balazipone or dosmalfate; free radical scavengers - WR 3689 (2-[[3-methylamino)propyl]amino]ethanediol dihydrogen phosphate ester, AD 20 (2-[[2-methoxyphenyl)acetyl]amino]-2-propenoic acid or nitroxide antioxidant; growth factors- granulocyte colony-stimulating factor (G-CSF), granulocyte-macrophage colony-stimulating factor (GM-CSF), Erythropoietin (EPO), epidermal growth factor (EGF), keratinocyte growth factor (KGF), transforming growth factor (TGF α and β), any interleukin including EL-11 and IL-15, insulin-like growth factor (IGF), nerve growth factor (NGF), platelet derived growth factor (PDGR), Bombesin, Relaxin, Calcitonin, colostrum-derived growth factor (CDGF), amlexanox or amoxanox, protegrin, pilocarpine hydrochloride, stem cell factor (STF), thrombopoietin, steel factor (SF), any interferon, including interferon or any cytokine.

In addition to cytoprotectants, the medicament may include one or more other pharmaceutical. compounds/agents. In particular, anti-microbial agents may be included. Such agents may be included to fight infections, for example secondary infections associated with mucositis, inflammatory bowel disease, irritable bowel syndrome etc.

The medicament manufactured according to any aspect of the invention is preferably administered by mouth or rectally (for ease of route to one or more parts of the alimentary canal) although parenteral administration of the medicament may also be used.

According to a fourth aspect of the invention, there is provided a composition comprising a lectin, for use in the control of mucosal cell proliferation, the reduction and/or treatment of damage caused by a cell-damaging agent, or a combination of two or more thereof. This aspect of the invention applies, in particular for lectins which have not previously been known to have a medical use. Relevant features of the first, second and third aspects of the invention also relate to the fourth aspect of the invention.

According to a fifth aspect of the invention there is a composition, comprising a lectin and a cytoprotectant. Such an aspect of the invention may relate to the first and/or second aspect of the invention, since it may be one embodiment of a medicament manufactured according to the first or second aspect of the invention. Accordingly, relevant features of the first and second aspects of the invention described above, also apply to the fifth aspect of the invention. A particularly preferred composition is one where the lectin is at least partially purified or isolated.

The composition of the fifth aspect of the invention may be a mixed preparation for administration, or may be a combined preparation for simultaneous, separate or sequential use (or administration). In the combined preparation, either the lectin or the cytoprotectant part of the composition may be administered fast.

The fifth aspect of the invention is particularly suitable for use in the reduction or treatment of damage caused by a cell-damaging agent, in particular, irradiation and/or chemotherapy. Accordingly, it may be appropriate to include in the composition pharmaceutically acceptable excipients and/or carriers as described according to the first and second aspects of the invention. As also described according to the first and second aspects of the invention, the composition is most effective against the biological matter which is most sensitive to cell-damaging agents, in particular, irradiation and chemotherapy.

All relevant features of aspects one to four of the present invention also apply to the fifth aspect.

A sixth aspect of the invention applies to a method for the manufacture of a composition according to the fifth aspect of the invention. The method may comprise admixing the lectin and the cytoprotectant, optionally in combination with one or more ingredients, such as additional cytoprotectants, anti-microbial agents and/or pharmaceutically acceptable excipients and/or carriers. Alternately, for a composition which has the different components administered simultaneously, separately or sequentially, the individual components are prepared which may be in combination with other ingredients, including pharmaceutically acceptable excipients and/or carriers.

For all therapeutic (alone) aspects of the invention (ie where the lectin is used only after any treatment, cell-damaging agent source etc.) the preferred dose of the lectin is less than 0.2g per kg body weight per day.

The present invention will now be illustrated by a number of non-limiting examples. The examples refer to the accompanying drawings, in which:
Figure 1 shows dry body weights of rats fed diets containing different amounts of phytohaemagglutinin for 10 days (Experiments 1 a, b, c). Fig 1a,; body dry weight vs mg PHA/day and Fig 1b relative body dry weight vs. log10 (mg PHA/day). (o, Experiment 1a; □ Experiment 1b; Δ Experiment 1c).
Figure 2 shows growth of rats fed on soya albumin diet followed by lactalbumin diet in repeated cycles in comparison with that of rats pair fed on control diet throughout the experiment. Times of switching the rats to different diets and food intakes are also indicated.
Figure 3 shows the effect of 5-FU and PHA administration on fresh body weight
Figure 4 shows the effect of 5-FU and PHA administration on daily food intake
Figure 5 shows the effect of lectin-containing diet on animal survival after 30 days, after receiving a 6.75 Gy dose of radiation.

### EXAMPLES

### Materials and Methods

### Purification of PHA

For the obesity and chemotherapy examples, PHA was purified by affinity chromatography on Sepharose 4B-fetuin using the method of Pusztai & Palmer (1977) with some improvements (Carvalho, 1993). Kidney beans were extracted with 0.05M sodium borate buffer (pH 8.0) and separated into globulins and albumins by dialysis against 0.033M sodium acetate buffer, pH 5.0. E-type PHA (erythroagglutinating) fractions were adsorbed on to Sepharose 4B-fetuin at pH 7.6 (0.05M Tris-HCl) and desorption with 0.05M glycine-HCl buffer, pH 3.0 also containing 0.5M NaCl, followed by dialysis and freeze-drying. For the purification of L-type (lymphoagglutinating) PHA, after the removal of small amounts of E-type PHA from the albumins by Sepharose 4B-fetuin, the non-absorbed fraction was fractionated on a sulphopropyl cation exchange HPLC column (TSK SP-5PW, 21.5 mm x 150 mm; Anachem GB Ltd) in 0.005 M sodium acetate-acetic acid buffer, pH 3.8 containing 0.1M NaCl and eluted by a programmed increasing ionic strength gradient (0.1-0.5M NaCl). Finally, lower molecular weight impurities were removed by chromatography on Sephadex G-100, and pure L-type PHA was recovered after dialysis and freeze drying. Recovery: 0.32 g and 0.61 g E-type and L-type PHA respectively per 100 g kidney bean meal.

For the radiotherapy example, PHA was isolated by grinding 50g of kidney beans in a grinder with a sieve of pore size 1 mm. 500ml of 0.02M acetic acid containing 0.1g ascorbic acid was added and stirred for 30 minutes at room temperature. The pH was adjusted to 5.0 with 1M NaOH and the solution stirred for a further 2 hours at room temperature. The solution was allowed to stand at 4 °C overnight and was then centrifuged at 9000 rpm for 15 minutes. 0.075g CaCl₂ was added to the supematent and the pH adjusted to 9.0 with 1M NaOH. The supernatant was allowed to stand again overnight at 4 °C and the sample was spun at 3000 rpm for 10 minutes. The sample was then dialysed against Tris (pH 7.6) before purification on a Fetuin-Sepharose 4B affinity column. The PHA peak was eluted with a 0.05M glycine buffer and then the PHA fraction was dialysed against water before freeze drying.

### Insulin assay.

Immunoreactive plasma insulin concentrations were measured using a double-antibody precipitation technique (MacRae *et al.,* 1991) and a rat insulin standard (Incstar Corporation, Stillwater, Min. USA). ¹²⁵I-labelled bovine insulin, 5 µCi/0.1 µg (ref. IM38) was supplied by Amersham International plc (Amersham, Bucks.) and antiserum to porcine insulin raised in guinea pigs by Miles Scientific (Stoke Poges, Slough). Anti-guinea pig IgG serum and normal guinea pig serum were from the Scottish Antibody Production Unit (Law Hospital, Carluke, Lanarkshire).

### Plasma glucose.

Concentration of glucose in plasma samples were carried out by the autoanalyzer method of Trinder (1967).

### Antibody production.

Antibodies to KTI, BBI, LA were developed in rabbits according to the method of Harboe and Inglid (1973) as described before (Hajos et al., 1995). Antibody to SBA was obtained from Sigma Chemical Co (UK Ltd).

### Competitive indirect ELISA.

Indirect ELISA assays were used for the quantitative determination of SBA in gut samples (Hajos *et al.,* 1995). However, with LA the ELISA plates were coated with LA and the immune-complex was formed by using a rabbit anti-LA IgG-type antibody. Results were expressed as per cent material recovered of the dose incubated intragastrically.

### Electrophoretic separation of antinutrients in gut samples.

SDS gel electrophoresis, followed by semi-dry transblotting on to nitrocellulose membranes and immunostaining with antigen-specific antibodies to the antinutrients were carried out as before (Hajos *et al.,* 1995).

### Composition of experimental diets

**Table 1**

| Composition of diets for obesity and chemotherapy examples. | | | |
|---|---|---|---|
| | Lactalbumin (LA) | Kidney Bean (KB) | KB Albumins (KBA) |
| Maize starch | 373 | 177 | 372 |
| Potato starch | 100 | 100 | 100 |
| Glucose | 150 | 150 | 150 |
| Corn oil | 150 | 150 | 150 |
| Vitamin mix | 50 | 50 | 50 |
| Mineral mix | 50 | 50 | 50 |
| Lactalbumin | 127 | 63 | 102 |
| Kidney bean meal | - | 260 | - |
| Kidney bean albumin | - | - | 26 |
| L-methionine | - | 2.1 | 0.9 |
| L-tryptophan | - | 0.25 | 0.13 |
| Silicic acid | 0.4 | 0.4 | 0.4 |

All components are given as g constituent/kg diet. For composition of vitamin and mineral mixes see Carvalho (1993).

**Table 2**

| Composition of diets for soybean anti-nutrient examples | | |
|---|---|---|
| Diet | 1. Control | 2. Soya albumins (SBALB) |
| Lactalbumin | 120 | 0 |
| Soyabean albumins | 0 | 110 |
| Maize starch | 380 | 390 |
| Potato starch | 100 | 100 |
| Glucose | 150 | 150 |
| Com oil | 150 | 150 |
| Vitamins | 50 | 50 |
| Minerals | 50 | 50 |
| L-tryptophan | 0 | 0.3 |
| L-methionine | 0 | 1.2 |
| L-phenylalanine | 0 | 1.0 |
| L-leucine | 0 | 2.3 |
| L-isoleucine | 0 | 2.6 |
| L-valine | 0 | 2.6 |
| Silicic acid | 0.4 | 0.4 |

All components are given as g constituent/kg diet. For composition of vitamin and mineral mixes see Grant *et al.* (1993).

**Table 3**

| Composition of diets for radiotherapy examples | | |
|---|---|---|
| Diet | 10% Lactalbumin | PHA (bean protein) |
| Lactalbumin | 120 | 90 |
| Kidney bean | 0 | 127.5 |
| Maize starch | 379.6 | 280.9 |
| Potato starch | 100 | 100 |
| Glucose | 150 | 150 |
| Corn oil | 150 | 150 |
| Vitamins | 50 | 50 |
| Minerals | 50 | 50 |
| L-tryptophan | 0 | 0.14 |
| L-methionine | 0 | 1.07 |
| Silicic acid | 0.40 | 0.40 |

All dietary components are given in g/kg diet. The PHA content of kidney bean is 2.6%. Accordingly, with a restricted daily dietary intake of 6 g, the input of PHA/mouse was 20 mg. Composition of vitamin mixture: Thiamine, 1000 mg; Pyridoxine (B6), 1000 mg; Riboflavin, 100 mg; p-amino benzoic acid, 1000 mg; Nicotinic acid, 3000 mg; Ca Pantothenate, 2000 mg; Folic acid, 500 mg; Biotin, 550 mg; Inositol, 40,000 mg; α-tocopherol, 25 g; Retinyl acetate, 1150 mg; Calciferol (D₃), 1500 mg; Vitamin B₁₂, 2.5 mg; Menadione, 500 mg; Choline chloride, 100 g; Maize starch, 5000 g.

### Administration of 5-FU

300mg of 5-fluorouracil was stirred in 14ml of distilled water. 1M NaOH was added slowly until the 5-FU had dissolved. The solution was made up to a final volume of 20 ml. The final pH of the solution was 8.3. A dose of 150 mg/kg body weight was administered to the animal by intraperitoneal injection. Immediately after the injection, the rats were offered 15 g of the control diet and food was available *ad-libitum* for the remainder of the experiment.

### Irradiation source

Cobalt ⁶⁰Co gun; 6.75 Gy total, full body exposure; dose rate: 0.3 Gy/min.

### Example 1.

Three separate experiments (1 a,b,c) were carried out to the same design. Rats weaned at 19 days were maintained on stock diet for 11 days and fed LA-diet for 3 days (Table 1) to reach 82-84 g starting weight. The rats were then selected into groups of five rats according to body weight and within each group they were allocated at random to treatment. Rats in each group were fed daily 6 g diet in the morning on a control, LA-diet or diets based on the LA-diet with different levels of PHA inclusion so that their daily PHA intake was between 0.65 and 42 mg/rat (0.007 and 0.45 g/kg body weight). After 10 days the rats were fed 2 g of respective diets in the morning and killed precisely 2 hours later. Gastrocnemius muscles were excised and rinsed and both bodies and muscles were freeze-dried and weighed. In Experiment 1 c bodies were ground to a powder and extracted with chloroform-methanol (2:1; v/v) for lipid determination.

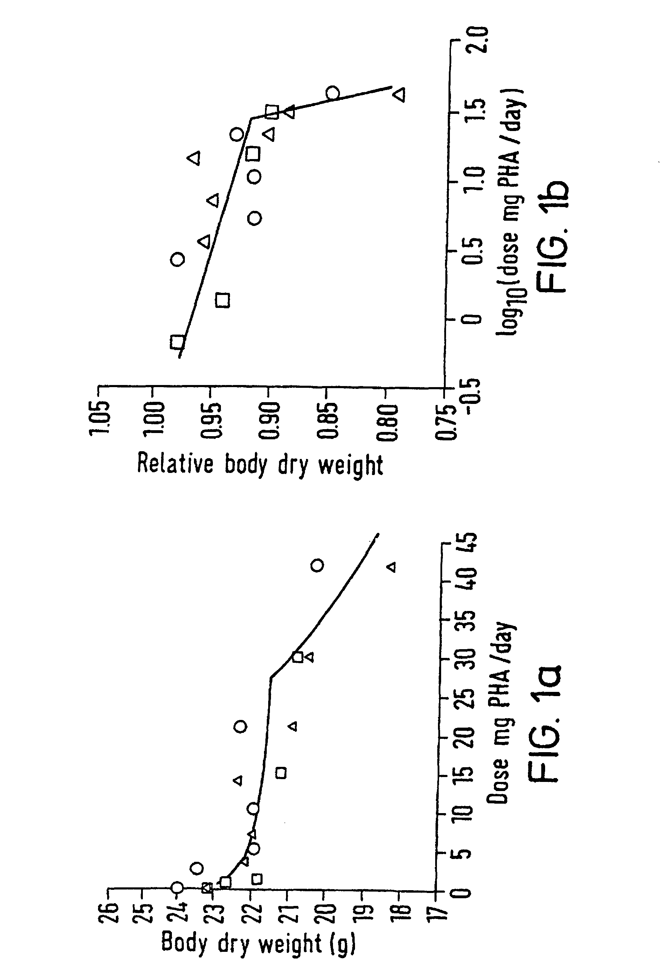

The mean body weights for the control groups in Experiments 1a,b,c were very similar lying between 23.2 and 24 g. Feeding rats with diets containing PHA in the range of 0 to 42 mg/rat/day (0 to 0.45 g/kg body weight) reduced their body weight in a biphasic manner (Figure 1a, b; Table 4, 5). There was a small reduction in body weight even at low levels of PHA (e.g. 4% at 3.5 mg/rat/d; 0.04 g/kg body weight) after which relatively large increases in the lectin dose (to about 0.32 g/kg) produced only modest further reductions in the body weight. Thus, averaged over all the experiments at doses below 10 mg PHA/d (0.12 g/kg body weight) the mean body weight reduction was 1.14 (se 0.25) g when compared to control (4.9% of control body weight). At daily doses of PHA between 10 and 27 mg (0.12 and 0.32 g/kg body weight) there was a further reduction of 0.64 (se 0.21) g (2.7% of control body weight). However, at higher doses (0.20 - 0.45 g/kg body weight) the reduction became more appreciable. The relationship between relative body dry weight (as proportion of zero dose control), RBDW, and the PHA dose expressed as mg/d from three separate feeding trials (Experiments 1 a, b, c) below PHA dose of 27 mg/d was:
RBDW = 0.918 (se 0.008) - 0.0334 (se 0.0062) x log10 (dose PHA/27)
Above this PHA dose the equation was:
RBDW = 0.918 (se 0.008) - 0.5138 (se 0.0876) x log10 (dose PHA/27)

Changes in the dry weight of the gastrocnemius (skeletal) muscles followed a similar trend with increasing lectin input (Table 4, 5). The proportional loss of muscle weight compared to control rats tended to be about 1.5 - 2.0 times that of the equivalent loss of body weight (Table 4, 5), but at daily doses of less than 10 mg PHA (0.12 g/kg body weight) the difference between the proportional loss of body and muscle weights was not significant (p > 0.05).

Similar to the reduction in body and muscle weights, the lipid content of the body of rats was reduced by increasing the dose of PHA in the diet (Table 5; Experiment 1c). However, proportionally the lipid loss exceeded that of both the body and skeletal muscle although the ratio of the losses remained roughly constant for all doses.

**Table 4**

| Body weights and gastrocnemius muscle weights of rats fed diets containing different levels of PHA for 10 days. | | |
|---|---|---|
| Dose of PHA mg/rat/day | Body dry weight (BDW) g (% loss vs control) | Gastrocnemius dry weight g (% loss vs control) |
| Experiment 1a | | |
| 0 | 24.0 - | 0.184 - |
| 2.6 | 23.5 2.2 | 0.183 0.7 |
| 5.2 | 21.9 8.7 | 0.166 9.7 |
| 10.5 | 22.0 8.5 | 0.155 15.5 |
| 21.0 | 22.4 7.0 | 0.153 16.5 |
| 42.0 | 20.4 15.2 | 0.129 29.8 |
| SED | 0.61 | 0.0074 |
| | | |

| Experiment 1b | | |
|---|---|---|
| 0 | 23.2 - | 0.178 - |
| 0.6 | 22.7 2.3 | 0.174 2.1 |
| 1.3 | 21.8 6.0 | 0.161 9.4 |
| 15.0 | 21.2 8.4 | 0.151 15.3 |
| 30.0 | 20.9 10.0 | 0.143 19.9 |
| SED | 0.35 | 0.0039 |

All doses were tested by 5 rats per group.

**Table 5**

| Body composition of rats fed diets containing different levels of PHA for 10 days (Experiment 1c). | | | | | | | | |
|---|---|---|---|---|---|---|---|---|
| PHA dose mg/rat/d | 0 | 3.5 | 7 | 14 | 21 | 30 | 42 | SED |
| Body dry weigh (g) | 23.2 | 22.2 | 22 | 22.4 | 21 | 20.6 | 18.4 | 0.47 |
| (% loss vs control) | - | 4.3 | 5.2 | 3.4 | 9.5 | 11.2 | 20.7 | |
| | | | | | | | | |
| Gastrocnemius dry weight (g) | 0.196 | 0.188 | 0.184 | 0.185 | 0.167 | 0.154 | 0.134 | 0.0061 |
| (% loss vs control) | - | 4.1 | 6.1 | 5.6 | 14.8 | 21.4 | 31.6 | |
| | | | | | | | | |
| Lipid weight (g) | 4.18 | 3.9 | 3.86 | 3.56 | 3.39 | 2.82 | 2.25 | 0.17 |
| (% loss vs control) | - | 6.7 | 7.7 | 14.8 | 18.9 | 32.5 | 46.1 | |
| | | | | | | | | |
| Total Muscle dry | 9.27 | 8.89 | 8.7 | 8.75 | 7.9 | 7.28 | 6.39 | |
| weight* (g) | | | | | | | | |
| | | | | | | | | |
| Other dry weight** | 9.75 | 10.39 | 9.46 | 10.14 | 9.69 | 10.46 | 9.75 | |
| (g) | | | | | | | | |
| | | | | | | | | |
| Ratio % loss of gastrocnemius vs % loss of lipid | - | 0.61 | 0.79 | 0.38 | 0.79 | 0.66 | 0.69 | |

| | | | | | | | | |
|---|---|---|---|---|---|---|---|---|
| Footnotes to Table 5. All groups had 5 rats per group. *TMDW was estimated based on assumptions (i) gastrocnemius dry weight/TMDW is same for all treatments and (ii) TMDW/BDW = 0.4 for the control group. | | | | | | | | |
| **OW was calculated by subtracting the sum of LW and TMSW from the total weight. | | | | | | | | |

PHA is widely regarded as a nutritional toxin because rats fed exclusively on kidney bean proteins containing high levels of PHA (0.8-1.0 g/kg body weight) die within a few days (Pusztai, 1991). However, as PHA is not harmful for germ-free rats its toxic effects in rats with a conventional microflora are likely to be the consequence of the dramatic *E. coli* overgrowth in the small intestinal lumen which was negligible below 0.2 g/kg but rose sharply and in proportion with the increase in the level of PHA in the diet (Pusztai et al., 1993). Example 1 shows that in this low concentration range where no bacterial overgrowth occurs (below 0.2 g/kg), the anti-nutritive effects of PHA are slight in rats harbouring a conventional microflora as the reduction in body weight is minimal after 10 days exposure to the lectin. Moreover, in contrast to muscle atrophy observed at high doses (0.45 g/kg and above), reduction in skeletal muscle weights below PHA doses of 0.10 g/kg body weight was slight and proportional to loss in final body weight (Tables 4, 5). However, relative to the control, the proportional loss of lipid was higher than the proportional loss of muscle although the ratio between them remained roughly constant (Tables 4,5). Thus the first effect of the lectin is a stimulation of body lipid catabolism and thus a low dose of lectin may be a suitable treatment for metabolic disorders such as obesity.

### Example 2

In Examples 2 - 5 the insulin response of rats to PHA was tested. In Example 2, rats were fed diets containing 42 mg PHA/day and blood insulin levels were measured after 9 and 10 days, respectively. Individually housed male Hooded Lister spf(specific pathogen-free) rats weaned at 19 days were maintained on stock diet (Special Diet Services, Manea, Cambridgeshire) for about 14 days *ad lib,* followed by restricted feeding (8 g/rat/d) for 5 days on a control, lactalbumin-based diet (LA; Table 1). Rats were fed three times daily; 2.5 g at 09.00 am, 1.0 g at 13.00 pm and 4.5 g at 18.00 pm. On the fifth day, the rats were given 1.5 g LA diet between 9.00 and 9.30 am and pre-experimental blood samples were taken from the tail vein 2 h later. Blood was collected in heparinized tubes (25 µl heparin solution containing 26 USP units/tube) and centrifuged in a bench-top centrifuge at + 1° C for 15 min. Plastic granules were used to aid the separation of plasma from erythrocytes; it was divided into 100 µl aliquots and stored at - 20° C until assayed. The rats were then randomly divided into two groups of 13 animals and individually housed. Group 1 was fed exclusively on a diet containing kidney bean (KB-diet; Table 1) for 10 days (8 g diet/rat/day; divided into 3 meals: 2.5 g at 09.00 am, 1 g at 13.00 pm and 4.5 g at 18.00 pm) while the control group was pair-fed on LA diet under the same conditions. On the 9th day, blood samples were taken in the morning exactly 2 h after the morning feed of 1.5 g of KB-diet and this protocol was repeated on the 10th (last) day. The rats were then killed under ether anaesthesia, the abdomen cut open and the rest of their blood was collected from the heart. The gastrointestional tract together with the pancreas was removed and after a quick rinse with ice-cold water, they were frozen in liquid nitrogen. Control rats were treated the same way except that they were given 1.5 g LA-diet before the blood samples were taken. Plasma samples were kept frozen till assayed for insulin.

Insulin was extracted from the pancreas (six randomly selected rats of each group) after homogenisation of a sample of this tissue (about 25-50 mg of dry weight) with 10 ml of acidified ethanol (ethanol: water: conc. H₂SO4 = 96 : 18 : 2.5; v/v/v) overnight in a cold room and then centrifuged at 1,500 g for 10 min in the cold. The clear supernatant was diluted to about 1 : 200 (v/v) with insulin assay buffer before including them in the insulin radioimmunoassay.

Feeding rats with KB containing 42 mg PHA/rat/day (0.45 g/kg body weight) for 10 days significantly reduced the plasma insulin concentration from the pre-experimental level of 2.97 (sd 0.84) ng/ml to 0.36 (sd 0.05) on the 9th day of the experiment (Table 6). The depression was apparently permanent during PHA exposure because blood samples taken on the 10th day were similarly low, 0.23 (sd 0.06) ng insulin/ml. In contrast, the plasma insulin levels in controls remained high, 3.31 (sd 0.30) and 1.55 (sd 0.21) ng/ml on the 9th and 10th days of the feeding respectively.

**Table 6**

| Weight and insulin content of pancreas of rats fed with diets containing kidney beans or lactalbumin (control) for 10 days. | | | | |
|---|---|---|---|---|
| | Pancreas (mg) | Dry Weight (mg/100g body weight) | µg/pancreas | Insulin µg/g protein |
| KB | 162 ± 20* | 982 ± 122* | 22,38 ± 7.63* | 182 ± 80* |
| LA | 138 ± 16 | 605 ± 63 | 40.60 ± 12.71 | 354 ± 152 |

| | | | | |
|---|---|---|---|---|
| KB = Kidney Bean Diet LA = Lactalbumin Diet Values are means ± SD for 6 animals in each group. *Significantly different from Lactalbumin-fed controls (P<0.01). | | | | |

The absolute and relative dry weights of the pancreas of rats fed on KB-diet at the highest dose of PHA for 10 days were significantly increased in comparison with pair-fed controls (Table 6). In contrast, the insulin content of the pancreas expressed either as µg/pancreas or µg/g protein was significantly decreased. Despite the highly significant reduction in insulin levels, plasma glucose concentrations were not significantly altered in KB-fed animals with an overall mean value of 1.7 (sd 0.1) mg glucose/ml for both treated and control rats.

The previously suggested link between the strong catabolic effects of high doses of PHA on body metabolism of lipids, carbohydrates and proteins and its lowering of plasma insulin levels (Pusztai, 1991) has now been confirmed. In fact, insulin levels were depressed not only in the blood circulation during the 10 day oral exposure of rats to PHA but also the insulin content of the pancreas was significantly reduced in these animals.

### Example 3

Rats were weaned at 19 days, kept in groups of 8-10 rats and fed on stock diet for 12 days. They were then randomly selected into two groups (13 rats in each group), individually housed for the rest of the experiment and fed on stock diet for another 8 days. Rats, after fasting overnight, were given 2 g stock diet in the morning and 2 h later blood-sampled (pre-experimental sample). Immediately after this the rats were intragastrically incubated with a 1 ml extract of KB (50 mg; 5-7 mg PHA) while the controls were dosed with 1 ml 0.01 M phosphate buffered saline (0.9% NaCl; w/v; PBS). Blood samples were obtained from each animal at 15, 60 and 120 min after the incubation. A single dose of a soluble KB protein sample caused a gradual decrease in plasma insulin. The pre-experimental value of 1.78 (sd 0.22) ng insulin/ml plasma decreased to 1.05 (sd 0.22) ng/ml after 120 min, i.e. some 59% of the initial value (Table 7). In control rats the insulin level remained roughly constant within experimental errors at all time points [1.76 (sd 0.42) ng/ml].

**Table 7.**

| Relative insulin levels (expressed as % of control) in rats after acute intragastric exposure to kidney bean proteins or purified E-type or L-type lectins | | | | |
|---|---|---|---|---|
| Time | Control | Kidney bean protein | E-type | L-type |
| 15 | 109 (36) | 82 (34) | 76 (27)* | 98 (32) |
| 60 | 97 (36) | 65 (21)* | 58 (23)* | 86 (34) |
| 120 | 89 (28) | 59 (15)* | 80 (39) | 81 (32) |

| | | | | |
|---|---|---|---|---|
| The results are means ± SD and * indicates that the mean is significantly different 100 (p<0.05). | | | | |

### Example 4

Example 4 was conducted in the same manner as example 3 except that the test animals were gavaged with a 1 ml solution of either 5 mg E-type or L-type lectins. Some of these lectin samples were labelled with ¹²⁵I (total counts of 2,5-3 million cpm). The controls received PBS. Blood samples were taken at 0, 15, 60 and 120 min as before. To measure the actual amounts of PHA delivered into the duodenum, radioactivity was measured in both stomach and small intestinal washes in some rats killed 1 or 2 h after incubation.

In rats incubated with pure E-type PHA, the pre-experimental plasma insulin levels were also decreased in the first 60 min to 1.03 (sd 0.32) ng/ml in a similar way to that found in animals gavaged with KB proteins (Table 7). However, there appeared to be a slight recovery in the next 60 min which rendered the change in insulin level at 120 min not significantly different (p > 0.05) from the pre-experimental value. A single dose of L-type PHA also appeared to cause a gradual reduction in plasma insulin but the changes were not significant at any of the time points during the 120 min of the experiment (1.39 sd 0.35 ng/ml at 120 min). The rates of stomach emptying in rats intubated with the lectins were slow and not significantly different for the two types of PHA (p > 0.05). With E-type about 52% of the initial dose reached the small intestine after 120 min, while with L-type PHA this was slightly more, about 63%. Plasma glucose levels were slightly reduced on acute exposure to PHA and/or KB albumins from 1.6 (sd 0.2) to 1.4 (sd 0.2) mg/ml but the reduction was not significant (p > 0.05).

A diet containing either pure E- or L-type PHA was able to exert a reduction in serum insulin as was seen with a diet containing kidney bean protein. Thus, the effect of reduced serum insulin is due to a direct effect of PHA and not due to the poor nutritional quality of the bean protein.

### Example 5

Sixteen rats weaned at 19 days and housed individually during the experiment were fed stock diet for 15 days, followed by 5 days on LA-diet (8 g diet/rat/day). Twelve randomly selected rats were then switched to a diet containing kidney bean albumin (KBA-diet; Table 1) for the next 3 days, with a daily intake of about 30 mg PHA/rat, while four control rats continued with the LA-diet. In the evening of the 3rd day, instead of the evening meal, the rats on KBA-diet were intragastrically intubated with a 1 ml solution of 100 mg of KBA sample containing 25 mg PHA, while the controls were given the evening portion of their LA-diet. After this the animals were not fed again till the following morning when they were all given 2 g LA-diet to boost their plasma insulin level. Exactly 2 hours later the rats were blood-sampled (pre-experimental sample), those which had been pre-fed on KBA were randomly selected into groups of four and were gavaged with 1 ml solutions of either 20 mg KBA, or 5 mg E-type or 5 mg L-type PHA lectins some of which were ¹²⁵I-labelled. The four control rats were gavaged with KBA (40 mg; 4-6 mg PHA) for comparison. Rats were blood sampled at 120 min, killed and pancreas removed and frozen for insulin assays.

Intragastric incubation with a single dose of purified PHA isolectins of rats pre-fed with diets containing KB albumin proteins or control diet and intubated with doses of PHA for 3 days substantially reduced the conceritration of plasma insulin in the circulation. With both E-type and L-type lectins the difference in insulin levels between pre-experimental [0.59 (sd 0.05) ng/ml] and 120 min values [(0.15 (sd 0.09) ng/ml] was significant (p > 0.05). Pre-feeding also appeared to speed up the rate of stomach emptying of intragastrically administered E-type lectins as over 80% of the dose reached the duodenum in the first 60 min. In contrast, L-type lectins were still slow to reach the duodenum and after the first 60 min about 50% of the initial amount of the PHA remained in the stomach. Despite the differences in the absolute plasma insulin concentrations between rats pre-fed on KBA-diet (low) and those kept on a control diet (moderately high), after gavaging with KB albumin proteins the proportional decrease in plasma insulin was similar in both groups of rats. The insulin content of the pancreas was slightly but not significantly reduced after the 3 days pre-feeding with KBA-diet from 58.3 (sd 10.8) in controls to 42.5 (sd 8.3) (g insulin/pancreas). However, there were no significant changes in the pre-experimental mean plasma glucose levels of about 1.6 (sd 0.2) mg/ml, although the values decreased slightly during the experiment. There were no significant changes in body and muscle weights after exposure to PHA for 3 days.

### Example 6

Dietary strategy for overcoming the antinutritional effects of soya by exploiting the increase in feed conversion efficiency after short periodic exposures of rats to soya whey having a high agglutinin content.

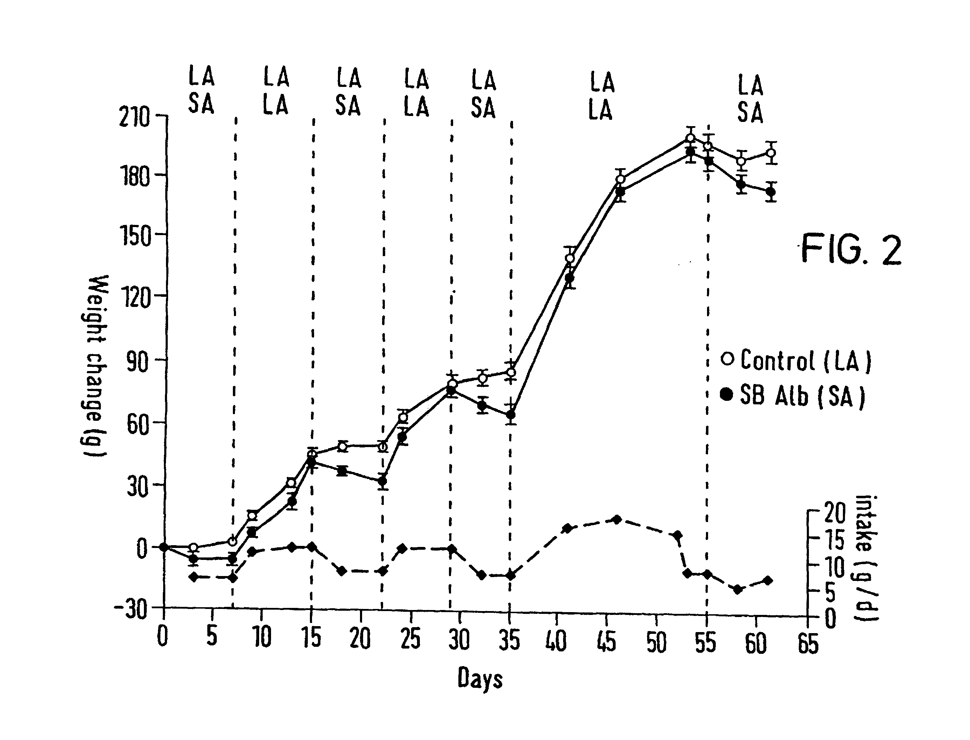

Male Hooded-Lister rats were weaned at 19 days and given free access to stock diet (Labsure, Manea, U.K.) for 7 days after which they were fed an LA control diet (100 g lactalbumin protein/kg; Table 2) *ad libitum* for 3 days, followed by feeding 6 g of the same diet/rat/d for 5 days. Water was freely available at all times. The rats were then divided into 2 groups, 5 rats in each group. The diet for the experimental group contained 100 g/kg protein based on SBALB (Table 2). The control group of rats were fed LA diet throughout the experiment and its amount was restricted to the voluntary intake of the test rats. The experimental design was such (Figure 2) that initially the soya group was fed soya diet for 7 days, switched to LA diet for 8 days, followed by soya diet for 7 days and a 7 day LA diet period respectively. Next, after another 6 days on soya diet followed by 20 days on LA diet, the rats were finally exposed to soya diet for a 5 day period. On the following morning which was the 61st day of the combined feeding experiment, all rats were given 2 g LA diet after which the soya group was intragastrically incubated with 280 mg SBALB dissolved in 2 ml saline while the controls received saline only. Rats were killed by halothane overdose exactly 90 min later and fully dissected. Stomach and small intestine were removed and the latter was cut into 10 cm long sections. The lumen of each tissue was washed with 2 ml ice-cold distilled water, freeze-dried and later reconstituted with distilled water (1 mg dry matter/100µl) and used for ELISA. A washed intestinal section of 2 cm (between 5-7 cm from the pylorus) was taken for histological examination. All tissues were freeze-dried and weighed. Rat bodies were also freeze-dried and used for the determination of protein and lipid contents. Stomach and small intestinal sections were homogenised (3 times) with 0.1 M D-galactose solution (5 ml/dry sample) and these extracts were used for ELISA. Throughout the experiment faeces were collected daily and used for nitrogen determinations.

In a control experiment the first cycle of the switching experiment was repeated but this time in addition to the SBALB group, a second set of test rats were first fed a diet containing LD-SBALB instead of SBALB for 7 days, followed by LA diet for 8 days while the control rats were pair-fed LA diet for the whole 15 days of the experiment. Weight gain, digestibility and feed conversion efficiency of the two test groups were compared with those of the control group in the two separate parts of the cycle.

The preparation used in the feeding experiment was shown by ELISA to contain 38.7 g SBA/kg. LD-SBALB contained less than 4 g SBA/kg. The weight of rats fed alternately on soya and LA diets was always significantly less at the end of each soya feeding period, including the last one, than that of the corresponding pair-fed control rats (Table 8 & 9). However, rats in the test group always grew faster in the LA diet period following soya feeding than control rats kept on LA diet throughout (Figure 2; Table 9). Moreover, feed conversion efficiency of the test group in the LA period was always significantly higher than that of the controls (Table 9).

**Table 8.**

| Rody weight (BW) and composition of rats | | |
|---|---|---|
| Diet | Control | Test(SBALB) |
| Initial BW (g) | 88.8 ± 3.5^{a} | 86.5 ± 2.1^{a} |
| Final BW (g) | 283.5 ± 7.5^{a} | 263.6 ± 7.2^{b} |
| Dry BW (g) | 111.5 ± 3.0^{a} | 104.6 ± 3.2^{b} |
| Lipid (g) | 54.1 ± 4.5^{a} | 51.8 ± 5.5^{a} |
| Protein (g) | 45.7 ± 1.5^{a} | 44.2 ± 2.1^{a} |
| Lipid | 485.2 ± 33.3^{a} | 493.8 ± 36^{a} |
| (g/kg Dry BW) | | |
| Protein | 410.7 ± 19.9^{a} | 402.1 ± 25.5^{a} |
| (g/kg Dry BW) | | |
| Results are given as means ± SD of 5 rats per group. Values in a horizontal row with distinct superscripts differ significantly (P≤0.05). | | |

**Table 9.**

| Weight changes and feed conversion efficiency of rats in test and control periods | | | |
|---|---|---|---|
| Treatment | Initial weight (g) | Final weight (g) | Feed conversion (g/g intake) |
| Test period Soya or LA diets | | | |
| **Switch 1 1-7d** | | | |
| Control | 88.8 ± 3.5^{a} | 92.2 ± 1.0 ^{a} | 0.08 ± 0.02 ^{a} |
| Test | 86.6 ± 2.1 ^{a} | 81.7 ± 3.2^{b} | negative ^{b} |

| **Switch 2 16-22d** | | | |
|---|---|---|---|
| Control | 133 ± 3.0 ^{a} | 137 ± 2.8 ^{a} | 0.06 ± 0.04 ^{a} |
| Test | 129 ± 3.0 ^{a} | 120 ± 3.9 ^{b} | negative ^{b} |

| **Switch 3 30-35d** | | | |
|---|---|---|---|
| Control | 168.5 ± 3.7 ^{a} | 174 ± 4.0 ^{a} | 0.11 ± 0.08 ^{a} |
| Test | 163.5 ±3.7 ^{a} | 153 ± 5 ^{b} | negative ^{b} |

| **Switch 4 56-61d** | | | |
|---|---|---|---|
| Control | 282.5 ± 6 ^{a} | 283.5 ± 6 ^{a} | 0.01 ± 0.08 ^{a} |
| Test | 281.6 ± 5.1 ^{a} | 263.6 ± 7.2 ^{b} | negative ^{b} |

| Control period (LA diet) | | | |
|---|---|---|---|
| **8-15d** | | | |
| Control | 92.2 ± 1 ^{a} | 133 ± 3 ^{a} | 0.43 ± 0.03 ^{a} |
| Test | 81.7 ± 3.2 ^{b} | 129 ± 3 ^{a} | 0.49 ± 0.03 ^{b} |

| **23-29d** | | | |
|---|---|---|---|
| Control | 137 ± 2.8 ^{a} | 168.5 ± 3.7 ^{a} | 0.38 ± 0.04 ^{a} |
| Test | 120 ± 3.9 ^{b} | 163.5 ± 3.7^{a} | 0.52 ± 0.04 ^{b} |

| **36-55d** | | | |
|---|---|---|---|
| Control | 174 ± 4 ^{a} | 282.5 ± 7.5 ^{a} | 0.36 ± 0.02 ^{a} |
| Test | 153 ± 5 ^{b} | 281.6 ± 5.1 ^{a} | 0.43 ± 0.02 ^{b} |

For each period values in a horizontal row with distinct superscripts differ significantly (P≤0.05).

In the soya feeding period the weight and nitrogen content of faeces of the test group was significantly higher than that of the control rats. However, in the LA periods there were no significant differences in these faecal values between rats in the test and control groups. Moreover, neither the lipid and protein contents nor their concentration in the rat bodies were significantly different in the two groups (Table 8).

Rats fed LD-SBALB in the test period gained more weight and had better feed conversion efficiency than those fed SBALB but their performance was still below that of rats fed LA diet (Table 10). However, rats switched from LD-SBALB to LA diet in the control period showed no significant improvement in feed conversion efficiency comparable to that with SBALB rats in the LA diet feeding part of the cycle (Table 10).

**Table 10.**

| Effect of lectin depletion on weight gain and feed conversion, | | | |
|---|---|---|---|
| Treatment | Initial weight (g) | Final weight (g) | Feed conversion (g/g intake) |
| **Test period Soya or LA diets** | | | |
| Control | 80.8 ± 1.5^{a} | 86.8 ± 2.0^{a} | 0.14 ± 0.03^{a} |
| SBALB | 80.6 ± 1.1^{a} | 78.7 ± 0.7^{b} | negative^{b} |
| LD-SBALB | 80.1 ± 1.1^{a} | 83 ± 1.1^{c} | 0.07 ± 0.03^{c} |
| | | | |

| **Control period (LA diet)** | | | |
|---|---|---|---|
| | | | |
| Control | 86.8 ± 2 ^{a} | 126.8 ± 2.5 ^{a} | 0.42 ± 0.03 ^{a} |
| SBALB | 78.7 ± 0.7 ^{b} | 126.7 ± 2.7 ^{a} | 0.50 ± 0.03 ^{b} |
| LD-SBALB | 83.0 ± 1.1 ^{c} | 124.2 ± 2.6 ^{a} | 0.43 ± 0.03 ^{a} |

For each period values in a horizontal row with distinct superscripts differ significantly (P≤0.05).

Effects on internal organs. The weights of stomach, small and large intestines, spleen, kidneys, thymus, lungs, heart and gastrocnemius muscles were not affected by feeding rats alternately with soya and LA diets for 61 days. However, the weight of the pancreas was significantly higher and that of the liver lower in the test group than in the controls.
In conclusion, diet-switching in which rats were fed alternately in short cycles on diets containing soya or lactalbumin has shown that it is possible to take advantage of the hyperplastic growth induced by SBA or other lectins to improve both absorption and utilisation of high-quality nutrients when the high-quality diet is fed. With this novel method processing of soya or other lectin-containing foodstuffs is not necessary and, moreover, soya waste products or other lectin-containing foodstuffs containing high amounts of lectin can be used (including in particular the whey fraction remaining after the removal of soya globulin proteins for many industrial uses).

### Example 7

The ability of orally ingested PHA to protect rats from a high dose of chemotherapy, and in particular its tissue protectant effect on the gut was investigated.

Four groups, each consisting of 5 rats were maintained on a precise dietary regime for a period of 7 days (Table 11).

**Table 11.**

| Chemotherapy dietary protocols | |
|---|---|
| Treatment | Dietary protocol |
| 1 | LA diet throughout, no 5-FU |
| 2 | LA 3d, inject 5-FU, LA 4d |
| 3 | PHA 3d, inject 5-FU, LA 4d |
| 4 | PHA 3d, inject 5-FU, PHA 3d, LA1d |
| Key LA = lactalbumin PHA *= Phaseolus vulgaris* agglutinin 5-FU = 5-Fluorouracil | |

Rats (approx 1 g) pre-dosed with PHA were offered 10 g of the control diet (Table 1) containing 10% lactalbumin. Each animal was given the equivalent of 20 mg PHA in 0.9% saline by gavage. Rats were offered control diet in two feeds at 1000 & 1700 hours. The amount of food given was strictly paired to the amount of diet eaten by the PHA pre-dosed animals. If the animals were immediately post-dosed with PHA, the lectin was administered two hours post 5-FU injection by gavage. Animals neither pre- or post dosed with PHA were administered with 1 ml 0.9% saline by gavage. After 3 days, animals in three of the groups were administered a dose of 150 mg/kg body weight 5-FU. Body weight of each animal was recorded daily and the average body weight calculated.

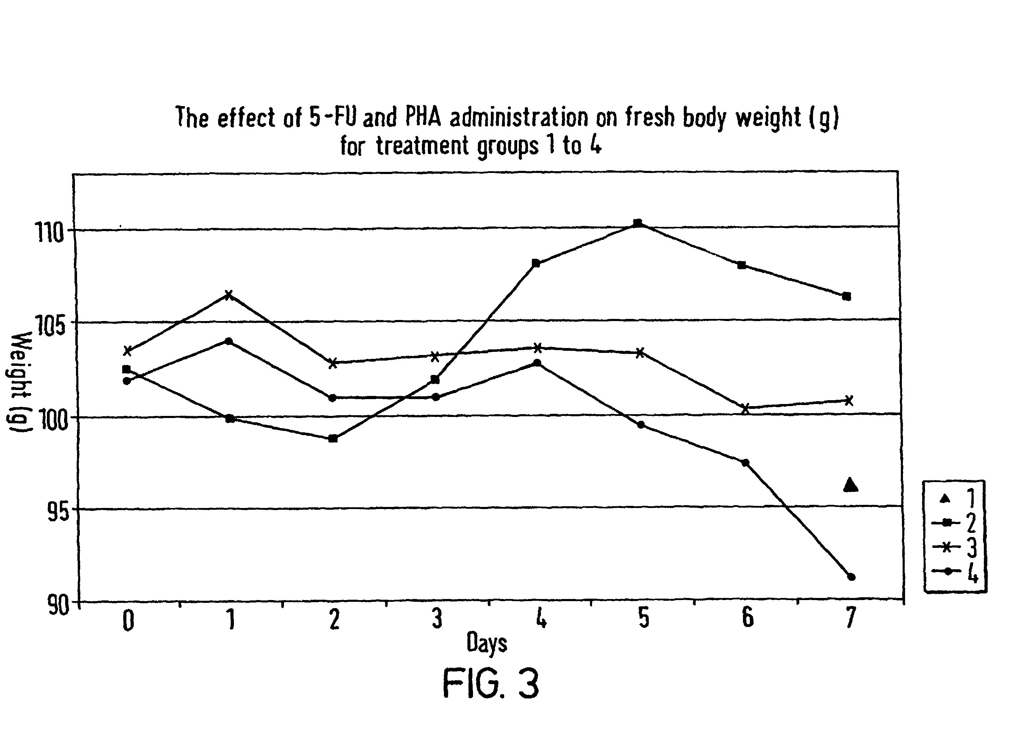

Figure 3 (see also Figure 3 data below) shows the effect of diet on animal body weight after administering 5-FU. Animals in the untreated control group grew at a steady rate throughout the experiment (data not shown). Animals maintained on the lactalbumin diet continued growing for 2 days after receiving the 5-FU diet before starting to lose weight. As this group was pair fed with the PHA pre-dosed group Whose intake is reduced by the presence of PHA in the diet, when food was re-introduced *ad lib* there was a compensatory increase in their food intake before the full cytotoxic effect of 5-FU took hold. Animals pre-dosed on PHA for three days before receiving 5-FU and fed on lactalbumin containing diet afterwards maintained a stable weight for the following four days and appeared normal. In the remaining treatment, the animals showed a 5-10% weight loss four days after the 5-FU dose.

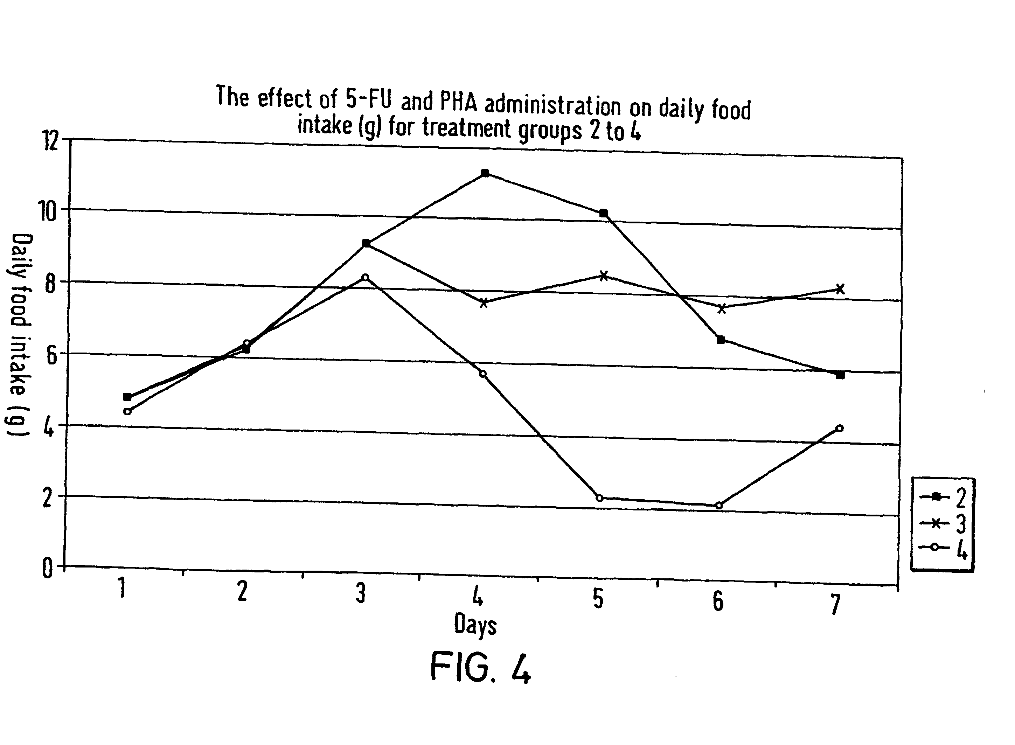

The food intake for each animal was recorded daily and the average food intake was calculated (Figure 4 and Figure 4 data below). In all treatments pre-dosed with PHA, the animals exhibited a steady increase in food intake prior to the 5-FU injection. Animals in the untreated control maintained a steadily increasing daily food intake (data not shown). Animals on the lactalbumin only diet reduced their food intake approximately one day after receiving the 5-FU dose. Animals pre-dosed on PHA for three days maintained a steady food intake at approximately 7 g/day for the four days following the 5-FU injection. The remaining treatment showed a large reduction in food intake for the four days following administration of 5-EU.

At the end of the experiment, the animals were sacrificed and then dissected. The dry weights of the major organs were recorded for each animal and the average weights calcurated. The average dry weights of the major organs of the gastrointestinal tract for each treatment are presented in Table 12.

### Figure data Appendix

Figure 3. The effect of 5-FU and PHA administration on fresh body weight (g).

| Treatment Group No. | | | | | | | | |
|---|---|---|---|---|---|---|---|---|
| **1** | nd | nd | nd | nd | nd | nd | nd | 96.2 |
| **2** | 102.5 | 99.9 | 98.8 | 101.9 | 108 | 110.2 | 107.9 | 106.2 |
| **3** | 103.5 | 106.4 | 102.8 | 103.2 | 103.6 | 103.3 | 100.3 | 100.7 |
| **4** | 101.9 | 104 | 101 | 101 | 102.76 | 99.5 | 97.4 | 91.2 |

Figure 4. The effect of 5-FU and PHA administration on daily food intake (g).

| Days | **1** | **2** | **3** | **4** | **5** | **6** | **7** |
|---|---|---|---|---|---|---|---|
| **1** | nd | nd | nd | nd | nd | nd | nd |
| **2** | 4.8 | 6.22 | 9.22 | 11.3 | 10.4 | 6.8 | 5.9 |
| **3** | 4.8 | 6.3 | 9.2 | 7.7 | 8.5 | 7.7 | 8.3 |
| **4** | 4.42 | 6.4 | 8.3 | 5.7 | 2.28 | 2.14 | 4.4 |

**Table 12.**

| The average dry weights (mg) of the major organs of the gastrointestinal tract after 7 days. | | | | | | |
|---|---|---|---|---|---|---|
| Treatment | Stomach | Small intestine | Jejunum | Ileum | Caecum | Colon |
| **1** | 141±6 | 887±28 | 185±1 | 151±8 | 124±4 | 147±6 |
| **2** | 140±7 | 509±55 | 90±8 | 94±11 | 99±11 | 130±11 |
| **3** | 136±13 | 840±253 | 189±37 | 109±24 | 114±29 | 143±24 |
| **4** | 125±6 | 759±318 | 168±70 | 91±24 | 93±18 | 117±21 |
| Key Treatments, see Table 11. | | | | | | |

The results show that administration of PHA before or after dosing with 5-FU had little effect on the stomach dry weight. However, PHA administration did have an effect on the small intestine dry weight If only lactalbumin was included in the diet, the small intestine was damaged by 5-FU and the dry weight was reduced by almost 50%. If however, PHA was administered for 3 days either directly before (Treatment 3), or directly before and after dosing with PHA (Treatment 4), the lectin was able to protect the small intestine from damage by 5-FU and the dry weights were similar to that of the control.

Within the small intestine, both the jejunum and ileum tissue were the most susceptible to damage by 5-FU (Table 12). However, if PHA was administered either directly before, or before and after the 5-FU injection, the lectin was able to exert a significant tissue protectant effect, particularly to the jejunum. Pre-dosing the animals with PHA three days before the 5-FU dose also gave a significant protective effect to the whole small intestine (Table 12). Administering PHA either directly before (Treatment 3) or directly before and after dosing with 5-FU (Treatment 4) gave the best tissue protectant effect. This result suggests that the dose of PHA administered is able to stimulate growth and repair of viable cells in the small intestine and provide protection against the cytotoxic effects of 5-FU.

After 7 days, blood was collected from the animals and both the key molecular and cellular components were analysed. The results are presented in Table 13.

**Table 13.**

| Analysis of key molecular and cellular components of blood from control and treated animals | | | | | | | | |
|---|---|---|---|---|---|---|---|---|
| Treatment | WBC no/mm³ | RBC no/mm³ | HGB g/100ml | HCT g/100ml | MCV µm³ | MCH pg | MCHC g/100ml | PLT no/mm³ |
| 1 | 5.5x10³ | 6x10³ | 12.1 | 34.2 | 57.5 | 20.4 | 35.4 | 552x10³ |
| 2 | 3x10³ | 7.4x10³ | 12.7 | 37.1 | 50.4 | 17.3 | 34.3 | 274x10³ |
| 3 | 2.4x10³ | 7.1x10³ | 13 | 34.7 | 48.7 | 18.2 | 37.4 | 296x10³ |
| 4 | 4x10³ | 7.4x10³ | 13 | 35.1 | 47.8 | 17.7 | 37.1 | 321x10³ |
| **Key** Treatments as Table 3 WBC White blood corpuscles RBC Red blood corpuscles HGB Haemoglobin HCT Haematocrit MCV Mean corpuscular volume MCH Mean corpuscular haemoglobin MCHC Mean corpuscular haemoglobin concentration PLT Platelets | | | | | | | | |

Following administration of 5-FU to rats fed the lactalbumin containing diet, the expected cytotoxicity was observed in both the number of white blood cells and platelets. None of the treatments had any significant effect on red blood cell count, haemoglobin & haematocrit content, mean corpuscular volume or haemaglobin concentration when compared to the untreated control. Administration of PHA directly before the 5-FU dose (treatment 3) had little effect on the measured parameters when compared to the lactalbumin only treatment (Treatment 2). Administration of PHA directly before and after the dose (Treatment 4) increased the number of white blood cells produced relative to the lactalbumin control.

The above results suggest that the relative timing of administering an oral dose of a lectin in relation to the cytotoxic drug may affect the haematological toxicity of the drug.

### Example 8

To identify a dose range at which pre-dosing with PHA protected the gastrointestinal tract from damage by 5-FU.

3 groups of rats (5 rats per group) were fed on the standard diet (Table 1) and gavaged daily with either 200, 100 & 50 mg/kg/day PHA for 3 days. The food intake for each rat was recorded daily. A second set of 3 control groups of rats (5 rats per group) were fed on the standard diet and gavaged daily with 1 ml saline for 3 days. Each animal of the control group was pair fed to an animal in the PHA dosed groups. On the morning of the 4^{th} day, each animal was injected with 150 mg/kg bodyweight 5-FU and then fed the standard diet for 6 days. 2 rats were fed on the standard diet *ad libitum* for 9 days. On the 9^{th} day, the rats were killed and the animals dissected. After freezing in liquid nitrogen, the wet tissue weights were recorded.

**Table 14.**

| The effect of pre-dosing with 200, 100 or 50 mg/kg/day PHA for 3 days on the jejunum and ileum wet weights (mg) after giving a dose of 150 mg/kg/BW 5-FU. | | |
|---|---|---|
| Treatment | Jejunum (mg) | Heum (mg) |
| Un-injected | 1006 | 909 |
| PHA 200 mg/kg/day | 1031 | 778 |
| Paired control 200 mg/kg/day | 848 | 753 |
| PHA 100 mg/kg/day | 984 | 800 |
| Paired control 100 mg/kg/day | 783 | 747 |
| PHA 50 mg/kg/day | 989 | 757 |
| Paired control 50 mg/kg/day | 761 | 722 |

By pre-dosing with 200 mg/kg/day PHA prior to injecting 5-FU, a significant protective effect was observed in the jejunum when compared to the un-injected treatment and paired control (Table 14). By reducing the dose of PHA to either 100 or 50 mg/kg/day, significant protection of the jejunum tissue was still observed when compared to the un-injected and pair control treatments. With the ileum tissue, PHA did not appear to exert such a profound tissue protectant effect for the doses examined when compared to the un-injected control. However, in every case, animals pre-dosed with PHA had larger ileal tissue than the corresponding pair fed controls.

The above examples demonstrate that chemotherapy can severely compromise the growth and viability of an animal. Manipulation of the diet, and in particular addition of a lectin before or after administering the cytotoxic drug can confer protection against chemotherapy. In particular, this protection is directed towards the viability and growth of the gastrointestinal tissues. The protection of gastrointestinal tissues after administering the cytotoxic drug was seen with doses of lectin of 200 mg/kg/day to 50 mg/kg/day.

### Example 9

The ability of orally ingested PHA to protect mice from a lethal dose of irradiation, and in particular its tissue protectant effect on the gut was investigated.

Eight groups, each consisting of 12 albino male mice were each irradiated with 6.75 Gy irradiation (0.3 Gy/min). Each group of mice was maintained on a precise dietary regime for a period of 30 days (Table 15).

**Table 15**

| Dietary protocols for the eight groups of mice used. | | |
|---|---|---|
| Mouse Group no. | Dietary Regime | |
| Irradiated treatment groups | | |
| 1 | SD 2d LA 3d SD 11d | *ir* LA 14d |
| 2 | SD 2d LA 3d | *ir* PHA 7d LA 7d SD 11d |
| 3 | SD 2d LA 1.5d FT1.5d | *ir* PHA 7d LA 7d SD 11d |
| 4 | SD 2d LA 2d PHA.1d | *ir* PHA 7d LA 7d SD 11d |
| 5 | SD 2d PHA 3d | *ir* PHA 7d LA 7d SD 11d |

| Controls | | |
|---|---|---|
| 6 | SD 2d LA 3d | LA 14d SD 11d |
| 7 | SD 2d LA 2d | PHA 1d PHA 7d LA 7d SD 11d |
| 8 | SD 2d PHA 3d | PHA 7d LA 7d SD 11d |
| Key to Table 15 SD Standard commercial diet (Charles River Ltd., Bioplan Ltd, Isaszeg, Hungary). LA Lactalbumin diet. A semi-synthetic diet with known composition shown in Table 1 (lectin free) formulated in the laboratory. The lactalbumin was obtained from Sigma (Poole, Dorset). PHA Kidney bean diet. A diet with known composition shown in Table 1 (containing the kidney bean lectin, PHA) formulated in the laboratory. The kidney bean source was cultivar 'Processor' (any other *Phaseolus vulgaris* bean-containing PHA is equally suitable). FT Fasting (no diet). *ir* Time of irradiation. | | |

The number of mice surviving after 30 days, and their average body weight were recorded.

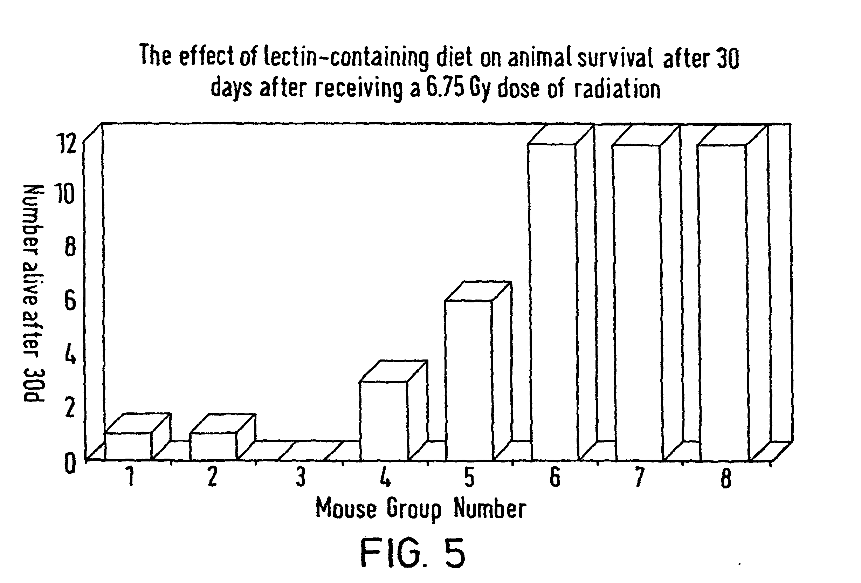

Figure 5 shows the effect of diet on animal survival after 30 days. Animals in the nonirradiated control treatments 6, 7 & 8 showed no effect of administering the standard commercial diet, lactalbumin diet and kidney bean diet on survival. No mortality was observed. Conversely, the animals in irradiated treatment groups 1, 2 & 3 with standard commercial, lactalbumin diets and the PHA diet administered after irradiation showed significant mortality (treatment 3 included a fasting period). Only a total of 2 animals in treatments 1-3 survived the irradiation treatment.

Where animals were fed on the PHA containing diet just before irradiation, (treatments 4 & 5) a significant increase in animal survival was observed. The number of surviving animals was closely correlated with the length of time animals were maintained on PHA diet directly prior to irradiation.

**Table 16**

| The Average Body Weight of Animals after 30 days. | |
|---|---|
| Mouse Group No. | Average body weight (g+/- standard deviation) |
| 1 | 29.9 |
| 2 | 29.6 |
| 3 | - |
| 4 | 23.0 +/- 5.63 |
| 5 | 30.12 +/- 1.83 |
| 6 | 31.88 +/- 2.02 |
| 7 | 31.18 +/- 1.46 |
| 8 | 31.03 +/- 2.11 |

After 30 days, animals in the control treatments 6-8 weighed 30-32g (Table 16). The animals in treatments 1-3 show high mortality. However, the animals in treatment groups 4 & 5 showed an increase in body weight correlated with the time animals were fed on the PHA containing diet prior to irradiation. In treatment group 5 (3 days on PHA containing diet prior to irradiation), the average body weight was similar to that of the control treatments. Administration of PHA into the diet at the dose studied had no detrimental effect. Where the lectin was administered before irradiation, a significant protective effect of PHA was observed.

Following irradiation of the animals, the average wet weights of small intestine, spleen and testicle were determined for each treatment (Table 17). Average weights for treatment group 7 were not determined.

**Table 17.**

| Small intestine, spleen and testicle weights (mg ± standard deviation) of surviving mice. | | | |
|---|---|---|---|
| Mouse Group No. | Small intestine | Spleen | Testicle |
| 1 | 999.8 | 72.5 | 49.4 |
| 2 | 1335.2 | 176.5 | 62.0 |
| 3 | - | - | - |
| 4 | 1032.0 ± 266.7 | 105.2 ± 25.7 | 53.6 ± 1.6 |
| 5 | 1312.3 ± 258.4 | 111.4 ± 37.4 | 62.8 ± 8.9 |
| 6 | 1477.3 ± 111.1 | 117.8 ± 13.5 | 208.0 ± 36.5 |
| 7 | nd | nd | nd |
| 8 | 1522.2 ± 159.3 | 107.8 ± 15.4 | 194.5 ± 18.8 |
| Key to Table 17 - indicates no data as no mice survived nd indicates no data recorded | | | |

These results show that testicular tissue was the most sensitive to the effect of irradiation. Administration of PHA prior or after irradiation had less effect on testicular growth.

Treatments 1-3, show low survival after irradiation. The results for treatments 4 & 5 suggest a dose dependent increase in small intestine weight correlated with the time the animals were maintained on PHA containing diet prior to irradiation. The average weight of the spleen tissue from treatments 4 & 5 was similar to that of the controls.

This example demonstrates that irradiation severely compromises animal viability and dietary manipulations can be used to modify the extent to which irradiation compromises viability. Fasting prior to irradiation does not confer protection against irradiation effects, indeed it may be detrimental. Dietary PHA had no detrimental effect on the parameters measured (groups 7 & 8 data compared to group 6) and testicular tissue, of those studied, was the most sensitive to irradiation damage, in terms of weight loss. In the example described, pre-dosing with PHA conferred protection against radiation damage, the degree of which appeared dependant on the time of PHA dosing.

### References

Archimund, E. & Thomas, X (1994). Administration of cytotoxic agents by continuous infusion in the therapy of acute myeloid leukemia. Journal of Infusional Chemotherapy, 4, 3-8.

Au, E., Koo, W.H., Tan, E.H. & Ang, P.T. (1996). A Phase II trial of etoposide, leucovorin and 5-Fluorouracil (ELF) in patients with advanced gastric cancer. Journal of Chemotherapy, 8, 300-303.

Bardocz, S., Brown, D.S., Grant, G., Pusztai, A., Stewart, J.C. & Palmer, RM. (1992). Effect of the β-adrenoreceptor agonist clenbuterol and phytohaemagglutinin on growth, protein synthesis and polyamine metabolism of tissues of the rat. British Journal of Pharmacology 106, 476-482.

Carvalho, A.F.F.U. de, (1993) Dietary kidney bean lectins affect insulin levels, change gene expression and modulate metabolism. Ph.D. thesis; University of Aberdeen.

Dieras, v & Pouillart, P. (1995). Infusional chemotherapy with new drugs: Taxanes, vinorelbine and topoisomerase I inhibitors. Journal of Infusional Chemotherapy, **5**, 191-192.

Denekamp, J. (1996). The broad spectrum of preclinical radiobiology: British contributions. International Journal of Radiation Oncology, Biology & Physics, 36, 497-509.

Erkisi, M., Erkurt, E., Ozbarlas, S., Burgut, R., Doran, F. & Seyrek, E. (1996). The use of recombinant human granulocyte colony-stimulating factor in combination with single or fractionated doses of isofamide and doxorubicin in patients with soft tissue sarcoma. Journal of Chemotherapy, 8, 224-228.

Grant, G., McKenzie, N.H., Watt, W.B., Stewart, J.C., Dorward, P.M. & Pusztai, A. (1986) Nutritional evaluation of soya beans (Glycine max): Nitrogen balance and fractionation studies. Journal of the Science of Food and Agriculture, **37**, 1001-1010.

Grant, G., Oliveira, J.T.A., de, Dorward, P.M., Annand, M.G., Waldron, M. & Pusztai, A. (1987). Metabolic and hormonal changes in rats resulting from consumption of kidney bean (*Phaseolus vulgaris*) or soyabean *(Glycine max).* Nutritional Reports International **36**, 763-772.

Grant, G., Dormand, P.M and Pusztai, A. (1993) Pancreatic enlargement is evident in rats Fed diets containing raw soybean (Glycine max) or cowpeas (Vigna unguiculata) but not those fed diets dased on kidney beans (Phaseolus vulgaris) or lupinseed (Luinas augustlfalinus). Journal of Nutrition **123** 2207-2215.

Gupta, Y.P. (1987) Nutritive value of soybean. International Journal of Tropical Agriculture, **5**, 247-279.

Hajos, Gy., Gelencser, E., Pusztai, A., Grant, G., Sakhri, M. & Bardocz, S. (1995) Biological effects and survival of trypsin inhibitors and the agglutinin from soybean in the small intestine of the rat. Journal of Agricultural and Food Chemistry, **43**, 165-170.

Harboe, N. & Inglid, A. (1973) Immunization, isolation of immunoglobulins, estimation of antibody titre. In A Manual of Quantitative Immunoelectrophoresis, Methods and Applications; (N.H. Axelsen, J. Kroll & B. Weeke, editors) Scandinavian Journal of Immunology, (Suppl. 1), pp. 161-164.

Isacoff, W.H., Frederick, R, Kuchenbecker, S.L., Jacobs, A.D. & Taylor, O. (1994). Continuous infusion 5-fluorouracil given with calcium luucovorin, dipyridamole, and Mitomycin-C in patients with advancer colerectal carcinoma: A Phase II trial. Journal of Infusional Chemotherapy, **4**, 107-111

Liener, I.E. (1994) Implications of antinutritional components in soybean foods. Critical Reviews in Food Science and Nutrition, **34**, 31-67.

Macrae, J.C., Bruce, L.A., Hovell, F.B. de B., Hart, I.C., Inkster, J., Walker, A. & Atkinson, T. (1991). Influence of protein nutrition on the response of growing lambs to exogenous bovine growth hormone. Journal of Endocrinology **130**, 53-61.

Palmer, RM., Pusztai, A., Bain, P. & Grant, G. (1987). Changes in rates of tissue protein synthesis in rats induced *in vivo* by consumption of kidney bean lectins. Comparative Biochemistry and Physiology **88C**, 179-183.

Paulsen, F., Hoffmann, W., Kortmann, R.D., Porschen, R. & Bamberg, M. (1996). Akute gastrointestinal Nebenwirkungen in der Radio-onkologie - Was ist gesichert in der Therapie?. Strahlenther. Onkol. 172,53-56 (Nr 2).

Podolsky, D.K. (1993). Regulation of intestinal epithelial proliferation: a few answers, many questions. Am. J. Physiol. **264**, pp G179-G186

Pusztai, A. (1991). Plant Lectins. Cambridge: Cambridge University Press.

Pusztai, A. & Palmer, R.M. (1977). Nutritional evaluation of kidney bean (*Phaseolus vulgaris*): the toxic principle. Journal of the Science of Food and Agriculture **28**, 620-623.

Pusztai, A., Greer, F. & Grant, G. (1989). Specific uptake of dietary lectins into the systemic circulation of rats. Biochemical Society Transactions **17**, 481-482.

Pusztai, A., Grant, G., Spencer, R.J., Duguid, T.J., Brown, D.S., Ewen, S.W.B., Peumans, W.J., Van Damme, E.J.M. & Bardocz, S. (1993). Kidney bean lectin-induced *Escherichia coli* overgrowth in the small intestine is blocked by GNA, a mannose-specific lectin. Journal of Applied Bacteriology **75**, 360-368.

Rackis, J.J., Wolf, W.J. & Baker, E.C. (1986) Protease inhibitors in plant foods; content and inactivation. In Nutritional and Toxicological Significance of Enzyme Inhibitors in Foods (M. Friedman, editor) Plenum Press, New York, pp. 299-331.

Sparano, J.A. & Wiernik, P.H. (1994). Infusional cyclophosphamide-based therapy for the treatment of lymphoma. Journal of Infusional Chemistry, **4**, 28-32.

Steel, G.G. (1996). From targets to genes: a brief history of radiosensitivity. Phys Med. Biol., **41**, 205-222.

Trinder, P. (1967). Determination of glucose in blood using glucose oxidase with an alternative oxygen acceptor. Annals Clinical Biochemistry **6**,24-27.

Van Halteren, H.K., Gortzak, E., Taal, B.G., Helmerhorst, Th, J, M., Aleman, B.M.P., Hart, A.A.M. & Zoetmulder, F.A.N. (1993). Surgical intervention for complications caused by late radiation damage of the small bowel: a retrospective analysis. European Journal of Surgical Oncology, **19**,336-341.

Yeoh, E., Horowitz, M., Russo, A., Muecke, T., Ahmad, A. & Chatterton, B. (1993). International Journal of Radiation Oncology, Biology & Physics, **26**, 229-237.

## Claims

1. Use of a lectin in the manufacture of a medicament for the reduction and/or treatment of damage to mucosal cells and/or tissues.

2. Use, as claimed in claim 1, for the reduction and/or treatment of gut lesions and/or mucositis.

3. Use, as claimed in claim 1, for the reduction and/or treatment of inflammatory bowel disease or irritable bowel syndrome.

4. Use, as claimed in any one of claims 1 to 3, for the reduction and/or treatment of damaged mammalian cells and/or tissue, in particular damaged human cells and/or tissue.

5. Use of a lectin as claimed in any one of claims 1 to 4 wherein the damage is caused by a cell-damaging agent.

6. Use, as claimed in any one of claims 1 to 5 wherein the cell-damaging agent is radiotherapy, a chemotherapeutic agent or a combination thereof.

7. Use of a lectin as claimed in any one of claims 1 to 6, in combination with a cytoprotectant, wherein the cytoprotectant is a radiosensitiser, a chemoprotectant, a growth factor or a combination of two or more thereof.

8. Use of a lectin as claimed in claim 7 wherein the cytoprotectant is one or more of vitamin K mimetics such as Synkavit or Menadione, gadolinium texaphyrin or iobenguane (([[3-iodo-13311)phenyl]methyl]guanidine): Sulcraphate, cysteine, cysteamine, Ethyol, balazipone or dosmalfate; free radical scavengers, WR 3689 (2-[[3-methylamino)propyl]amino]ethanediol dihydrogen phosphate ester, AD 20 (2-[[2-methoxyphenyl)acetyl]amino]-2-propenoic acid or nitroxide antioxidant; granulocyte colony-stimulating factor (G-CSF), granulocyte-macrophage colony-stimulating factor (GM-CSF), Erythropoietin (EPO), epidermal growth factor (EGF), keratinocyte growth factor (KGF), transforming growth factor (TGF α and β), any interleukin including IL-11 and IL-15, insulin-like growth factor (IGF), nerve growth factor (NGF), platelet derived growth factor (PDGR), Bombesin, Relaxin, Calcitonin, colostrum-derived growth factor (CDGF), amlexanox or amoxanox, protegrin, pilocarpine hydrochloride, stem cell factor (STF), thrombopoietin, steel factor (SF), any interferon, including interferon or any cytokine.

9. Use of a lectin, as claimed in any one of claims 1 to 8, wherein the medicament administers a lectin in a concentration of from 0.3g to 0.1µg per kg body weight per day.

10. Use of a lectin as claimed in any one of claims 1 to 9 wherein the lectin is kidney bean, soya bean, jack bean, wheat germ, lotus seed, onion, lentil, tomato, potato or combinations of two or more thereof.

11. Use of a lectin as claimed in any one of claims 7 to 10 in combination with an anti-microbial agent.

12. A pharmaceutical composition comprising a lectin and a cytoprotectant selected from a radiosensitiser, a chemoprotectant, a growth factor or combinations thereof.

13. A pharmaceutical composition as claimed in claim 12 for use in the reduction and/or treatment of damage to mucosal cells and/or tissues.

14. A composition as claimed in any one of claims 12 to 13 for simultaneous, separate or sequential use in the prevention or treatment of damage caused by a cell-damaging agent.

15. A composition, as claimed in any one of claims 12 to 14, wherein the lectin is kidney bean, soya bean, jack bean, wheat germ, lotus seed, onion, lentil, tomato, potato or combinations of two or more thereof.

16. A composition as claimed in any one of claims 12 to 15 further comprising an anti-microbial agent.

## Patentansprüche

1. Verwendung eines Lektins bei der Herstellung eines Medikamentes zur Verringerung und/oder zur Behandlung eines Schadens von Schleimhautzellen und/oder Geweben.

2. Verwendung nach Anspruch 1 zur Verringerung und/oder Behandlung von Darmläsionen und/oder Schleimhautentzündung.

3. Verwendung nach Anspruch 1 zur Verringerung und/oder die Behandlung von entzündlichen Darmkrankheiten oder Reizdarmsyndrom.

4. Verwendung nach einem der Ansprüche 1 bis 3 zur Verringerung und/oder Behandlung von geschädigten Säugetierzellen und/oder geschädigtem Gewebe, insbesondere vom geschädigten menschlichen Zellen und/oder Gewebe.

5. Verwendung eines Lektins nach einem der Ansprüche 1 bis 4, wobei der Schaden durch ein zellschädigendes Mittel verursacht ist.

6. Verwendung nach einem der Ansprüche 1 bis 5, wobei das zellschädigende Mittel Strahlentherapie, ein chemotherapeutisches Mittel oder eine Kombination davon ist.

7. Verwendung eines Lektins nach einem der Ansprüche 1 bis 6 in Kombination mit einem Zellschutzmittel, wobei das Zellschutzmittel ein Strahlensensitivierer, ein chemoprotektives Mittel, ein Wachstumsfaktor oder eine Kombination von zwei oder mehreren davon ist.

8. Verwendung eines Lektins nach Anspruch 7, wobei das Zellschutzmittel ein oder mehrere Vitamin-K-Mimetika ist, wie beispielsweise Synkavit oder Menadion, Gadolinium-Texaphyrin oder Iobenguan (([[3-Iodo-13311) phenyl]methyl]guanidin): Sulcraphat, Cystein, Cysteamin, Ethyol, Balazipon oder Dosmalfat; freie Radikalfänger, WR 3689 (2-[[3-Methylamino)propyl]amino] ethandioldihydrogenphosphatester, AD 20 (2[[2-Methoxyphenyl)acetyl]amino]-2-propansäure oder Nitroxid-Antioxidans; Granulozyten-Kolonie-Stimulierender-Faktor (G-CSF), Granulozyten-Makrophagen-Kolonie-Stimulierender-Faktor (GM-CSF), Erythropoietin (EPO), epidermaler Wachstumsfaktor (EGF), Keratinozyten-Wachstumsfaktor (KGF), transformierender Wachstumsfaktor (TGF α und β), jedes Interleukin, einschließlich IL-11 und IL-15, insulinähnlicher Wachstumsfaktor (IGF), Nervenwachstumsfaktor (NGF), von Blutplättchen abgeleiteter Wachstumsfaktor (PDGR), Bombesin, Relaxin, Calcitonin, aus Kolostrum abgeleiteter Wachstumsfaktor (CDGF), Amlexanox oder Amoxanox; Protegrin, Pilocarpinhydrochlorid, Stammzellfaktor (STF), Thrombopoietin, Steel factor (SF), jedes Interferon, einschließlich Interferon, oder jedes Zytokin ist.

9. Verwendung eines Lektins nach einem der Ansprüche 1 bis 8, wobei das Medikament ein Lektin in einer Konzentration von 0,3 g bis 0,1 µg pro kg Körpergewicht pro Tag verabreicht.

10. Verwendung eines Lektins nach einem der Ansprüche 1 bis 9, wobei das Lektin aus der Gartenbohne, Sojabohne, Jackbohne, Weizenkeimen, Lotussetzlingen, Zwiebeln, Linsen, Tomaten, Kartoffeln stammt oder aus Kombinationen von zwei oder mehreren davon.

11. Verwendung eines Lektins nach einem der Ansprüche 7 bis 10 in Kombination mit einem antimikrobiellen Mittel.

12. Pharmazeutische Zusammensetzung, umfassend ein Lektin und ein Zellschutzmittel, ausgewählt aus einem Strahlensensitivierer, einem chemoprotektiven Mittel, einem Wachstumsfaktor oder Kombinationen davon.

13. Pharmazeutische Zusammensetzung nach Anspruch 12 zur Verwendung bei der Verringerung und/oder bei der Behandlung eines Schadens von Schleimhautzellen und/oder Geweben.

14. Zusammensetzung nach einem der Ansprüche 12 bis 13 für simultane, einzelne oder aufeinander folgende Verwendung in der Prävention oder Behandlung von Schäden, verursacht durch ein zellschädigendes Mittel.

15. Zusammensetzung nach einem der Ansprüche 12 bis 14, wobei das Lektin aus der Gartenbohne, Sojabohne, Jackbohne, Weizenkeimen, Lotussetzlingen, Zwiebeln, Linsen, Tomaten, Kartoffeln oder Kombinationen von zwei oder mehreren davon stammt.

16. Zusammensetzung nach einem der Ansprüche 12 bis 15, die weiters ein antimikrobielles Mittel enthält.

## Revendications

1. Utilisation d'une lectine dans la fabrication d'un médicament pour la réduction et/ou le traitement d'un dommage aux cellules et/ou aux tissus de muqueuse.

2. Utilisation suivant la revendication 1, pour la réduction et/ou le traitement de lésions de l'intestin et/ou d'une inflammation de muqueuse.

3. Utilisation suivant la revendication 1, pour la réduction et/ou le traitement d'une maladie inflammatoire de l'intestin ou du syndrome de l'intestin irritable.

4. Utilisation suivant l'une quelconque des revendications 1 à 3, pour la réduction et/ou le traitement de cellules et/ou d'un tissu endommagés de mammifère, en particulier des cellules et/ou un tissu endommagés humains.

5. Utilisation d'une lectine suivant l'une quelconque des revendications 1 à 4, dans laquelle le dommage est provoqué par un agent endommageant les cellules.

6. Utilisation suivant l'une quelconque des revendications 1 à 5, dans laquelle l'agent endommageant les cellules est une radiothérapie, un agent chimiothérapeutique ou une combinaison de ceux-ci.

7. Utilisation d'une lectine suivant l'une quelconque des revendications 1 à 6, en combinaison avec un agent cytoprotecteur, dans laquelle l'agent cytoprotecteur est un agent radiosensibilissant, un agent chimioprotecteur, un facteur de croissance ou une combinaison de deux de ceux-ci ou davantage.

8. Utilisation d'une lectine suivant la revendication 7, dans laquelle l'agent cytoprotecteur est un ou plusieurs parmi des substances mimétiques de la vitamine K comme le Synkavit ou la Ménadione, la texaphyrine de gadolinium ou l'iobenguane (([[3-iodo-13311)phényl]méthyl]guanidine) : le Sulcraphtate, la cystéine, la cystéamine, l'Ethyol, la balazipone ou le dosmalfate; des piégeurs de radicaux libres, le WR 3689, l'ester dihydrogénophosphate de (2-[[3-méthylamino)-propyl]amino]éthanediol, l'AD 20, l'acide (2-[[2-méthoxyphényl)acétyl]amino]-2-propénoïque ou l'antioxydan nitroxyde; le facteur stimulant la formation de colonies de granulocytes (G-CSF), le facteur stimulant la formation de colonies de granulocytes et de macrophages (GM-CSF), l'érythropoïétine (EPO), le facteur de croissance épidermique (EGF), le facteur de croissance des kératinocytes (KGF), le facteur de croissance transformant (TGF α et β), une quelconque interleukine notamment l'IL-11 et l'IL-15, le facteur de croissance de type insuline (IGF), le facteur de croissance des nerfs (NGF), le facteur de croissance dérivé des plaquettes (PDGR), la Bombésine, la Relaxine, la Calcitonine, le facteur de croissance dérivé du colostrum (CDGF), l'amlexanox ou l'amoxanox, la protégrine, le chlorhydrate de pilocarpine, le facteur des cellules souches (STF), la thrombopoïétine, le facteur Steel (SF), un quelconque interféron, notamment l'interféron ou une quelconque cytokine.

9. Utilisation d'une lectine suivant l'une quelconque des revendications 1 à 8, dans laquelle le médicament administre une lectine en une concentration de 0,3 g à 0,1 µg par kg de poids corporel par jour.

10. Utilisation d'une lectine suivant l'une quelconque des revendications 1 à 9 dans laquelle la lectine est de haricot sec, de soja, de haricot sabre, de germe de blé, de graine de lotus, d'oignon, de lentille, de tomate, de pomme de terre ou des combinaisons de deux de celles-ci ou davantage.

11. Utilisation d'une lectine suivant l'une quelconque des revendications 7 à 10, en combinaison avec un agent antimicrobien.

12. Composition pharmaceutique comprenant une lectine et un agent cytoprotecteur sélectionné parmi un agent radiosensibilisant, un agent chimioprotecteur, un facteur de croissance ou des combinaisons de ceux-ci.

13. Composition pharmaceutique suivant la revendication 12, pour une utilisation dans la réduction et/ou le traitement d'un dommage à des cellules et/ou des tissus de muqueuse.

14. Composition suivant l'une quelconque des revendications 12 à 13, pour une utilisation simultanée, distincte ou séquentielle dans la prévention ou le traitement d'un dommage provoqué par un agent endommageant les cellules.

15. Composition suivant l'une quelconque des revendications 12 à 14, dans laquelle la lectine est de haricot sec, de soja, de haricot sabre, de germe de blé, de graine de lotus, d'oignon, de lentille, de tomate, de pomme de terre ou des combinaisons de deux de celles-ci ou davantage.

16. Composition suivant l'une quelconque des revendications 12 à 15, comprenant en outre un agent antimicrobien.
